# EUROPEAN PATENT APPLICATION

(11) **EP 2 093 236 A1**
(43) Date of publication of application: **26.08.2009**
(21) Application number: 07817383.8
(22) Date of filing: 30.10.2007
(51) Int. Cl.: C07K 16/22

(54) **RECOMBINANT ANTIBODIES AGAINST VASCULAR ENDOTHELIAL GROWTH FACTOR (VEGF)**

(30) Priority: 01.11.2006 CU 20060208
(71) Applicant: Centro De Ingenieria Genetica Y Biotecnologia, Ciudad De La Habana 10600 (CU)
(72) Inventor: LAMDAN ORDÁS, Humberto, Bauta, La Habana 32400 (CU); GAVILONDO COWLEY, Jorge, Víctor, Ciudad De La Habana 10 400 (CU); AYALA AVILA, Marta, Ciudad De La Habana 11 600 (CU); ROJAS DORANTES, Gertrudis, Ciudad De La Habana 10500 (CU); MORERA DÍAZ, Yanelys, Ciudad De La Habana 13545 (CU); GUIROLA CRUZ, Osmany, Ciudad De La Habana 10300 (CU); CHINEA SANTIAGO, Glay, Ciudad De La Habana 11 600 (CU); SANTIAGO VISPO, Nelson, Francisco, Ciudad De La Habana 11 600 (CU)
(74) Representative: Hatzmann, Martin
(86) International application number: PCT/CU2007/000019
(87) International publication number: WO 2008/052489

(57) **Abstract**

The present invention deals with recombinant polypeptide molecules related to antibodies, that specifically recognize the human Vascular Endothelial Growth Factor A (VEGF-A), and interfere with its *in vitro* stimulatory effects and pro-angiogenic activity *in vivo*. These recombinant polypeptide molecules affect proliferation of human endothelial cells *in vitro,* subcutaneous angiogenesis in mice induced by Matrigel pellets that contain VEGF-A and the growth of human tumors transplanted in nude athymic mice. Several of these moleculas prevent choroideal neovascularization in a non human primate experimental model. These molecules can be employed for passive immunotherapy in pathological entities which have in its base an abnormal increase in blood vessels, as: age-related macular degeneration (wet variant), cancer and its metastases, neovascular glaucoma, diabetic and newborn retinopathies, acute and chronic inflammatory processes, infectious diseases, autoimmune diseases, organ transplant rejection, hemangioma, angiofibroma, and others.

## Description

### Technical field

The present invention is related with the field of biotechnology and pharmaceutical industry, in particular with the development and application of recombinant polypeptide molecules related to antibodies, that recognize specifically the human Vascular Endothelial Growth Factor A (VEGF-A) (Ferrara, N. et al. 2003. Nature Medicine 9: 669-676) and that interfere with its *in vitro* stimulatory effects and *in vivo* pro-angiogenic activity. Among these molecules are a single chain Fv antibody fragment (scFv), a Fab antibody fragment, and "full antibody" type bivalent molecules (scFv₂-Fc).

### Background of the invention

The process of formation of new blood vessels from pre-existing ones is denominated angiogenesis, and is regulated through the equilibrium of pro- and anti-angiogenic factors. Among the diseases that have been related with the anomalous induction of pro-angiogenic factors and the formation of new blood vessels are: cancer (primary tumors and its metastases), acute and chronic inflammatory processes as asthma, respiratory distress, endometriosis, atherosclerosis and tissue edema, diseases of infectious origin as the hepatitis and Kaposi sarcoma, autoimmune diseases as diabetes, psoriasis, rheumatoid arthritis and thyroiditis, and several other diseases and states such as diabetic retinopathy, organ transplant rejection, age-related macular degeneration (wet variant), neovascular glaucoma, hemangioma, and angiofibroma (Carmeliet, P. y Jain, RK. 2000. Nature 407: 249-257; Kuwano M, et al. 2001. Intern Med 40: 565-572).

An attractive therapeutic procedure for many of these diseases is based in the inhibition of the activity of pro-angiogenic factors that stimulate anomalous formation of vessels, through the administration of neutralizing molecules. In medical practice, the recombinant humanized antibody Bevacizumab, known commercially as Avastin (Ferrara, N. et al. 2005. Biochem Biophys Res Comun 333: 328-335), that recognizes and neutralizes the pro-angiogenic effect of human VEGF-A, has been approved in several countries for the treatment of tumor processes. It has been shown that its effect mainly depends on the inhibition of the VEGF-A induced neo-angiogenesis produced by the tumor cells and others of the tumor stroma, such as macrophages and fibroblasts. This antibody was originally obtained as a murine monoclonal antibody (Kim, KJ. et al. 1992. Growth Factors 7: 53-64) through the immunization of mice with the isoform 165 of human VEGF-A obtained from mammalian cells. The antibody was then modified by genetic engineering to achieve its humanization, that confers the molecule a better tolerance and therapeutic efficacy when applied to humans.

Recently, Ranibizumab (Gaudreault, J. et al. 2005. Invest Ophthalmol Visual Sci 46: 726-733), an antibody fragment derived from the already mentioned Avastin and commercially known as Lucentis, has been approved in several countries for the treatment of age-related macular degeneration (wet variant). Ranibizumab is a recombinant Fab antibody fragment originated from the manipulation of Bevacizumab using genetic engineering. The intravitreous injection of Ranibizumab neutralizes the locally-produced VEGF-A and controls further neo-angiogenesis of the retina, that is the basis of the disease.

The vascular endothelial growth factors are a family of molecules that induce in a direct and specific manner the formation of new blood vessels (Leung, D. et al. 1989. Science 246:1306-1309). This family comprehends the Vascular Permeability Factor (VPF), also known as Vascular Endothelial Growth Factor, now denominated VEGF-A, the Placental Growth Factor (PIGF), the Platelet-Derived Growth Factors (PDGF) PDGF-A and PDGF-B, and other VEGF-A structurally and functionally related molecules denominated VEGF-B, VEGF-C, VEGF-D, and VEGF-E (Olofsson, B. et al. 1996. Proc Natl Acad Sci USA 93: 2576-2581; Joukov, V. et al. 1996. EMBO J 15:290-298; Yamada, Y. et al. 1997. Genomics 42:483-488; Ogawa, S. et al. 1998. J Biol Chem 273:31273-31282).

VEGF-A is a homodimeric glycoprotein formed by two 23 kDa subunits (Ferrara, N. et al. 1989. Biochem Biophys Res Comun 161: 851-858) of which 5 monomer isoforms exist, derived from the differential splicing of one same ribonucleic acid (RNA). These include two isoforms that remain attached to the cell membrane (VEGF 189 and VEGF 206) and three of soluble nature (VEGF 121, VEGF 145, and VEGF 165). VEGF 165 is the most abundant in mammalian tissues, exception made of lung and heart, where VEGF 189 is higher (Neufeld G et al. 1995. Canc Met Rev 15:153-158), and in placenta, where VEGF 121 prevails (Shibuya, M. 1995. Adv Cancer Res 67: 281-316).

VEGF-A is the most studied and characterized protein of this family, and its alteration has been described in a larger number of diseases. VEGF-A over expression is associated with tumors of different origin and localization as well as its metastases (Grunstein, J. et al. 1999. Cancer Res 59: 1592-1598), chronic inflammatory processes as ulcerative colitis and Chron's disease (Kanazawa, S. et al. 2001. Am J Gastroenterol 96: 822-828), psoriasis (Detmar, M. et al. 1994. J Exp Med 180: 1141-1146), respiratory distress (Thickett, DR. et al. 2001. Am J Respir Crit Care Med 164: 1601-1605), atheroesclerosis (Celletti, FL. et al. 2001. Nat Med 7: 425-429), endometriosis (McLaren, J. 2000. Hum Reprod Update 6: 45-55), asthma (Hoshino, M. et al. 2001. J Allergy Clin Immunol 107: 295-301), rheumatoid arthritis and osteoarthritis (Pufe, T. et al. 2001. J Rheumatol 28: 1482-1485), thyroiditis (Nagura, S. et al. 2001. Hum Pathol 32: 10-17), diabetic and newborn retinopathy (Murata, T. et al. 1996. Lab Invest 74: 819-825; Reynolds, JD. 2001. Paediatr Drugs 3: 263-272), macular degeneration and glaucoma (Wells, JA. et al. 1996. Br J Ophthalmol 80: 363-366), tissue edema (Kaner, RJ. et al. 2000. Am J Respir Cell Mol Biol 22: 640-641), obesity (Tonello, C. et al. 1999. FEBS Lett 442: 167-172), hemangiomas (Wizigmann, S. y Plate, KH. 1996. Histol Histopathol 11: 1049-1061), in the sinovial liquid of patients with inflammatory artropathy (Bottomley, MJ. et al. 2000. Clin Exp lmmunol 119:182-188), and associated to transplant rejection (Vasir, B. et al. 2001. Transplantation 71: 924-935). In the particular case of tumors, the cells that express the three basic isoforms of VEGF-A (121, 165 and 189) are the ones that grow faster *in vivo* (Grunstein, J. 2000. Mol Cell Biol 20: 7282-7291).

The alterations in the function of endothelial cells induced by molecules of the VEGF family are mediated by their binding to tyrosine kinase class 3 receptors, that until now include VEGFR1 (Flt1), VEGFR2 (KDR/Flk1) and VEGFR3 (Flt4) (Kaipainen, A. 1993. J Exp Med 178: 2077-2088). The second N-terminal domain of the receptors has been identified as the responsible for ligand binding that favors phosphorilation of the cytoplasmic domain and signal translation (Davis-Smyth, T. et al. 1996. EMBO 15: 4919-4927).

VEGFR2 (KDR/Flk1) mediates the biological effects of VEGF-A, and also binds VEGF-C, and VEGF-D. This receptor is expressed differentially in the active endothelium and in several cell lines of tumor origin, where autocrine-like loops of stimulation may establish with the secreted VEGF. On top of being involved in the aforementioned pathologies, that are related with the over-expression of its ligands, receptor over-expression has been also associated with the progression of: endometrial cancer (Giatromanolaki, A. et al. 2001. Cancer 92: 2569-2577), malignant mesothelioma (Strizzi, L. et al. 2001. J Pathol 193: 468-475), astrocytic tumors (Carroll, RS. et al. 1999. Cancer 86: 1335-1341), breast primary cancer (Kranz, A. et al. 1999. lnt J Cancer 84: 293-298), intestinal-type gastric cancer (Takahashi, Y. et al. 1996. Clin Cancer Res 2: 1679-1684), glioblastoma multiforme, anaplastic oligodendroglioma and necrotic ependimoma (Chan, AS. et al. 1998. Am J Surg Pathol 22: 816-826). KDR over-expression has been also associated with autonomic VHL disease and hemangioblastoma (Wizigmann-Voos, S. et al. 1995. Cancer Res 55:1358-1364), the progression of diabetc retinopathy (Ishibashi, T. 2000. Jpn J Ophthalmol 44:323-324). Together with Flt-1, KDR has been associated with delayed hypersensitive reaction (Brown, LF. et al. 1995. J Immunol 154:2801-2807)

The majority of the new therapeutic strategies involving the inhibition of angiogenesis, especially in cancer, are based on the blockade of VEGF-A and/or its receptors. Standing out among the approved products or those in clinical trials we find the following: (1) monoclonal antibodies that block VEGF-A or KDR, (2) metaloproteinase inhibitors, as Neovastat and Prinomastat, (3) VEGF inhibitors as Talidomide, Suramin, Troponin I, IFN-α and Neovastat, (4) VEGF receptor blockers as SU5416, FTK787 and SU6668, (5) inducers of apoptosis of tumor endothelium as Endostatine and CA4-P, and (6) ribozymes that lower VEGF expression or that of its receptors (Angiozyme).

Of all the aforementioned, antibodies (and antibody fragments) that neutralize the pro-angiogenic effects of VEGF-A are the ones that have most advanced regarding application and acceptance as therapeutic products. Additionally to the examples of Bevacizumab and Ranibizumab that were mentioned above and have been already registered, there are other reports that mention antibodies and antibody fragments that recognize and neutralize human VEGF (Muller, Y. et al. 1997. Proc Natl Acad Sci USA 94: 7192-7197; Asano, M. et al. 1998. Hybridoma 17:185-190; Vitaliti A. et al. 2000. Cancer Res 60: 4311-4314; Brekken, RA. y Thorpe, PE. 2001. J Controlled Release 74:173-181; Jayson, G. et al. 2002. JNCI 94: 1484-1493; Brekken, RA. et al. 2000. Cancer Res 60: 5117-5124; Fuh, G. et al. 2006. J Biol Chem 281: 6625-6631; US 5730977).

### Detailed description of the invention

The present invention describes recombinant polypeptide molecules related to antibodies that comprehend human immunoglobulin variable regions (VRs) encoded by the nucleotide sequences SEQ ID No. 7 and SEQ ID No. 8, -or homologous sequences-, that recognize an epitope in human VEGF-A defined over, while not necessarily limited to, residues C102, C57, R56, T31 and L32, and that interfere with VEGF-A pro-angiogenic effect. The ability of such molecules to neutralize the pro-angiogenic effects of human VEGF-A in *in vitro* and *in vivo* models relevant to neo-angiogenesis is demonstrated. These molecules are formed by one or more antigen binding sites, such sites constituted by the aminoacids encoded by the DNA sequences of human immunoglobulin heavy and light chain VRs.

To the effects of this invention, the following terminology is defined:
• Recombinant antibodies
   Describes an immunoglobulin or parts thereof produced in part or fully by synthetic means (by way of recombinant DNA or artificial gene synthesis) with specific recognition of an antigen through one or more interaction domains (formed by particular combinations of immunoglobulin heavy and light chain variable regions, that are commonly denominated antigen binding sites) (Gavilondo y Larrick. 2000. Biotechniques 29: 128-136). Examples of recombinant antibodies are the so-called chimeric and humanized antibodies, in which the variable regions (or parts thereof), obtained from one specie, are associated using genetic engineering with immunoglobulin constant regions of another species. Among recombinant antibodies we also have antibody fragments produced by genetic engineering that comprehend one or more antigen binding sites. Examples of recombinant antibody fragments are: (i) Fab fragments, that include the VL, VH, CL and CH1 of an immunoglobulin; (ii) Fd fragments, consisting of VH and CH1 domains; (iii) Fv fragments, formed by the VL and VH of an antibody; (iv) scFv fragments, where the VH and VL domains of an antibody are linked in different form (VH-VL or VL-VH) through a peptide linker segment that allows the association of the two domains to form a antigen binding site (Bird et al. 1988. Science 242: 423-426; Huston et al. 1988. PNAS USA 85: 5879-5883); (v) "diabodies", multivalent or multi-specific fragments constructed in a similar manner to scFv, but where the small size of the linker does not allow the VH and VL domains of a single scFv molecule to associate with each other, and the binding sites have to be formed through the association of two or more individual scFv molecules (WO94/13804; Holliger P et al. 1993. PNAS USA 90: 6444-6448); (vi) dAb (Ward SE et al. 1989. Nature 341: 544-546), isolated complementarity determining regions (CDR), F(ab')₂ fragments, and scFv bispecific dimers (PCT/US92/09965; Holliger P y Winter G. 1993. Current Opinion Biotechnol. 4: 446-449; de Haard, H et al. 1998. Adv. Drug Delivery Rev. 31:5-31). Some types of fragments, as scFv and Fab, can be also obtained from antibody libraries, where a wide repertoire of VR genes (synthetic or derived from natural sources) of a specie are combined at random to produce particular associations of antibody VR, that are displayed later in the surface of filamentous phage.
   "Antibody-type" molecules where antibody fragments are artificially assembled with antibody constant regions by genetic engineering are also considered recombinant antibodies. For example, it is possible to construct a bivalent "antibody-type" molecule by joining a scFv to a region formed by the hinge, CH2, CH3, and sometimes CH4 domains of an immunoglobulin Fc. Depending on the availability of all or several of the described regions and the presence of glycosylation, the antibody-type molecule can exhibit effector functions commonly associated to immunoglobulin Fc.
• Antigen binding site. Epitope
   The first term describes the portion of an antibody that interacts specifically with an antigen (or part thereof). When the molecular size of the antigen is large, the antibody may bind to only a particular zone of the antigen, which zone is denominated epitope. An antibody binding site is formed mainly by two antibody variable regions, the light chain variable region, and the heavy chain variable region. The antibody binding site is formed by the non covalent interaction of the variable regions. The antibody binding site can be stabilized artificially through the linkage of the two variable regions with a linker peptide that will not interfere with its properties of specifically recognizing the antigen, as is the case of a scFv fragment. In nature, the antibody binding sites are assembled through the non covalent interaction of variable regions, that is reinforced through the non covalent interaction of the CH1 and CL (kappa or lambda) domains that follow in the heavy and light chain variable regions, respectively, of the molecule's native structure. Native full antibodies possess two identical antigen binding sites. The epitope recognized by an antibody binding site, in the case in which the antigen is a protein, can be formed by a linear sequence of aminoacids, or can be conformational, that is, the aminoacids recognized by the antibody binding site are close in the tertiary structure of the protein, but are not necessary sequential in its primary structure. In the case of proteins, the epitope is naturally a discreet zone, defined by a particular group of aminoacids that interact with the antibody by non covalent linkage.
• Homologous antibody
   It is a natural or genetic engineering-produced antibody that recognizes specifically the epitope of an antigen that is also specifically identified by another and different antibody. The two antibodies in question can be related in terms of the sequences of their variable regions (for example, one derives from another due to mutations that can be more or less extensive), or can have completely different variable region sequences. The latter is due to the fact that the specific interactions between antibodies and antigens, and specially in the case of proteins, is done through surface interactions, that is, the formation of noncovalent bonds (hydrogen bonds, van der Waals and similar) between aminoacid residues of the variable regions (this is considering that some other discreet residues structurally close to the variable regions can sometimes also participate in the interaction). This produces two different antibodies with distinct binding sites in terms of sequence but that can nonetheless have enough identity of interaction with a particular epitope that makes both specific for it. A homologous antibody has then to be able to identify specifically the particular epitope recognized by other antibody. The term homologous is extensive to other forms of antibodies comprehended in the definitions made above for recombinant antibodies (antibody fragments, "antibody-type" molecules, and others).
• Specific
   Refers to the situation in which an antibody or antibody fragment does not show a significant binding to other molecules different from its specific binding partner. This term is also applicable to the case where an antigen binding site is specific for a particular epitope that appears in a number of related or unrelated antigens, in which case the binding site will be able to bind several antigens that possess the mentioned epitope.

In one embodiment of this invention, the recombinant polypeptide molecules related to antibodies are: a human antibody fragment (scFv 2H1) of the single chain Fv type (scFv), a Fab antibody fragment (Fab 2H1-32) and scFv₂-Fc bivalent molecules of the "full-antibody" type (scFv₂-Fc 2H1 4.1 and scFv₂-Fc 2H1 8.2). In all of these the different VRs assemble spontaneously to form antigen binding sites. To the stability of this assembly contribute artificial linkage segments (linkers) or other antibody related sequences, as immunoglobulin constant domains. The VRs derive from those contained in a scFv that was isolated from a library of human antibody fragments displayed in the surface of filamentous phage, constructed using a VR repertoire of human lambda chains.

Due to the employed strategy, the recombinant polypeptide molecules related to antibodies described in this invention have immunoglobulin VRs with novel DNA sequences and different to those reported by other authors that have also obtained antibodies that neutralize the pro-angiogenic action of VEGF-A, as those derived from: hybridomas (Kim, KJ. et al. 1992. Growth Factors 7:53-64; Muller, Y. et al. 1997. Proc Natl Acad Sci USA 94: 7192-7197; Asano, M. et al. 1998. Hybridoma 17:185-190; Schaeppi, JM. et al. 1999. J Cancer Res Clin Oncol 125: 336-342; Brekken, RA. et al. 2000. Cancer Res 60: 5117-5124; Brekken, RA. y Thorpe, PE. 2001. J Controlled Release 74:173-181), viral transformation of human cells (US 5730977), modification of pre-existing antibodies by genetic engineering (Jayson, G. et al. 2002. JNCI 94: 1484-1493; Ferrara, N. et al. 2005. Biochem Biophys Res Comun 333: 328-335), and human antibody fragment libraries (Vitaliti, A. et al. 2000. Cancer Res 60: 4311-4314; Fuh, G. et al. 2006. J Biol Chem 281: 6625-6631). The recombinant polypeptide molecules related to antibodies described in this invention are also novel in the sense that they recognize a conformational epitope in human VEGF-A that is different from those defined by other antibodies that neutralize human VEGF-A (Muller, Y. et al. 1997. Proc Natl Acad Sci USA 94: 7192-7197; Muller, AY. et al. 1998. Structure 6: 1153-1167; Schaeppi, JM. et al. 1999. J Cancer Res Clin Oncol 125: 336-342; Brekken, RA. et al. 2000. Cancer Res 60: 5117-5124; Fuh, G. et al. 2006. J Biol Chem 281: 6625-6631; WO2005012359).

The anti-angiogenic effects obtained with the application of the recombinant polypeptide molecules related to antibodies described in this invention, and their equivalent variants, are based on the interference of the interaction between human VEGF-A and its receptors present in activated vascular endothelial cells, thus affecting the capacity of the latter to proliferate and maintain their physiological stability.

In the context of this invention, "equivalent variants" are polypeptide molecules derived from other associations and manipulations of the sequences contained in the VRs 2H1RVCP (SEQ ID No. 7) and 2H1RVCL (SEQ ID No. 8) and other homologous VRs contained in this invention (SEQ ID No. 13) that retain the capacity of specifically recognizing human VEGF-A and interfering with its biological effect of stimulation of growth of endothelial cells, and pro-angiogenesis. These polypeptide molecules can take the form of other recombinant antibody fragments, as an scFv where the VL domain is prior to the VH domain in the sequence, or where other union segments (linkers) known in the state of the art are used to join the VRs, or as F(ab')2, Fabc, Facb, dimeric scFv, trimeric scFv, and tretrameric scFv antibody fragments (Winter G, Milstein C. 1991. Nature 349: 293-299; WO94/13804; de Haard, H et al. 1998. Adv. Drug Delivery Rev. 31:5-31). Also equivalent variants are produced after the addition of other sequences derived from immunoglobulins, to form multivalent molecules (Bestagno M et al. 2001. Biochemistry 40: 10686-10692). "Equivalent variant" molecules can also be in the form of bispecific antibodies, where a portion of the molecule preserves its specificity for VEGF-A and the other has a different specificity, or in the form of full antibodies, where the VRs sequences are associated to immunoglobulin constant regions of human or other origin. All these manipulations using genetic engineering are known for those skilled in the art.

"Equivalent variants" are also considered those molecules produced through the so-called "CDR transplant" in which the CDR sequences contained in the VRs 2H1RVCP (SEQ ID No. 7) and 2H1RVCL (SEQ ID No. 8), and other homologous VRs as those contained for example in SEQ ID No. 13, are artificially flanked by sequence frameworks that are not the original, as is revealed for example in EP-B-0239400, EP-A-184187, GB 2188638A or EP-A-239400, in a way this manipulation will not affect the capacity of specifically recognizing human VEGF-A and of interfering with the growth of endothelial cells and pro-angiogenesis.

The recombinant polypeptide molecule in the form of scFv fragment (denominated scFv 2H1) recognizes specifically different isoforms of human VEGF-A. In the scFv, the human immunoglobulin heavy and light chain VRs are genetically associated in this order by a 16 aminoacid linker, to form the DNA sequence described in SEQ ID No 6. The scFv 2H1 fragment was obtained from an homologous scFv denominated scFv 2H1-F, selected from a library of scFv fragments displayed in filamentous phage constructed by methods similar to those already described (Rojas G, et al. 2005. Biochem Biophys Res Comun 336:1207-1213), using a repertoire of human lambda chain VRs. The intentional bias introduced in the light chain VR repertoire was done to increase the possibility of finding antibodies different to those reported by other authors, and that would neutralize VEGF-A through mechanisms different to those previously identified.

In the process of selection from the library we employed a fusion protein that contains the isoform 121 of human VEGF-A mutated in residues R82, K84, H86 by their substitution for glutamic acid and that affects its interaction with the KDR receptor (Shen, B. et al. 1998. J Biol Chem 273: 29979-29985), as shown in Example 1. This recombinant fusion protein (denominated P64₄₇ₐₐ-VEGF, SEQ ID No. 3) was produced in bacteria, and purified in a form similar to that employed for molecular species with the *in vitro* biological activity of human VEGF-A. In consequence, the antigen used in this invention is different from antigens or immunogens used by other authors that have obtained monoclonal or recombinant antibodies that recognize human VEGF. The P64K protein domain of *Neisseria meningitidis*, localized in the N-terminal end of P64₄₇ₐₐ-VEGF, augments immunogenicity, and allows high level expression in *E. coli* and the formation of dimeric forms similar to human VEGF with biological activity. The mutated zone comprehends an epitope already recognized by other antibodies reported as neutralizing of the biological functions of VEGF-A (Muller, Y. et al. 1997. Proc Natl Acad Sci USA 94: 7192-7197; Muller, AY. et al. 1998. Structure 6: 1153-1167).

In agreement with this invention, the use of the fusion protein P64₄₇ₐₐ-VEGF for the selection of binding sites for human VEGF from a library of scFv antibody fragments that contains a lambda chains VR repertoire was a determinant factor in the identification of an antibody fragment (scFv 2H1-F) that recognizes a conformational epitope of human VEGF-A not previously described.

To produce the polypeptide molecule scFv 2H1, the DNA sequence of the scFv 2H1-F fragment obtained from the library was cloned from the correspondent phagemide vector into a periplasm expression vector. The scFv 2H1 antibody fragment, with an apparent molecular weight somewhat higher than 29 kDa, is recovered from the culture medium and periplasm of the transformed bacteria, and easily purified using metal ion affinity chromatography (IMAC) (Porath J. 1992. Prot. Expr. Purif. 3: 263-281). The expression vector pACR.1 adds to the C-terminus of the molecule scFv 2H1 a c*-myc* peptide domain that is used as "tag" for analytical purposes, followed by a six-histidine domain to facilitate the purification using IMAC.

The recombinant polypeptide molecule in the form of a Fab fragment (denominated Fab 2H1-32) recognizes specifically different isoforms of human VEGF-A. In the Fab, the DNA sequences encoding of the VRs of scFv 2H1, denominated 2H1RVCP (SEQ ID No. 7) and 2H1 RVCL (SEQ ID No. 8) were cloned in the vector pFabHum-1, and genetically associated to the CH1 and Cλ of a human IgG-type immunoglobulin, respectively, as described in SEQ ID No. 10 and SEQ ID No. 9. The pFabHum-1 plasmid is a bicistronic vector constructed for the expression of Fab fragments with Cλ and CH1 human constant regions in the bacterial periplasm. The produced polypeptide chains comprehend the VRs and their respective light or heavy constant region, that are assembled to form a Fab fragment in the bacterial periplasm, to where they are exported from the cytoplasm through the signal peptides provided by the vector. The periplasmic assembly of the Fab is based in non covalent interactions between the light and heavy chain VRs, and between the CH1 and Cλ domains, but it is reinforced through a covalent bond of the disulfide type between two cysteines located close to the C-terminus of the CH1 and Cλ regions, also provided by the vector. The Fab 2H1-32 has an apparent molecular size of 50 kDa.

The recombinant polypeptide bivalent molecules of the "full antibody" type denominated scFv₂-Fc 2H1-8.2 and scFv₂-Fc 2H1-4.1 comprehend, respectively, the VRs sequences of scFv 2H1, followed by a sequence that encodes for ten spacer aminoacids, a nucleotide sequence that encodes for the hinge, CH2, and CH3 domains of a human immunoglobulin of the IgG1 type (SEQ ID No. 14), and a sequence (SEQ ID No 13) very similar to the previous, with limited changes in some VRs bases. These molecules were obtained after the cloning of the PCR products of the gene that encodes for scFv 2H1, excluding its 3' domains (that encode for the c-*myc* peptide and six histidines), in the pVSJG-HucFc vector. The pVSJG-HucFc vector is designed for the expression in mammalian cells of "full antibody" type molecules that comprehend two identical scFv associated to the Fc of a human IgG1 immunoglobulin. The polypeptide chain that gives way to these bivalent molecules is transported to the endoplasmic reticulum of the mammalian cells via an immunoglobulin signal peptide present in the pVSJG-HucFc vector. The peptide is removed in the endoplasmic reticulum and two identical polypeptide chains covalently associate through disulfide bonds in the hinge regions, a link that is reinforced by non covalent interactions of the CH2 and CH3 regions. The scFv₂-Fc 2H1-4.1 and scFv₂-Fc 2H1-8.2 molecules have a similar apparent molecular size between 100 and 120 kDa. These secreted proteins have in their amino-terminus four additional aminoacids (QVLK) provided by the vector.

The recombinant antibodies of this invention, like scFv 2H1, Fab 2H1-32, scFv₂-Fc 2H1-4.1 and scFv₂-Fc 2H1-8.2, can also be produced in other eukaryotic systems, as is the case of transgenic plants (Pujol, M. et al. 2005. Vaccine 23: 1833-1837).

In other aspect of this invention, the recombinant polypeptide molecules described above recognize specifically the isoforms 121 and 165 of human VEGF-A, and do not identify mouse VEGF. The molecules can bind to soluble human VEGF-A, to human VEGF-A adsorbed in solid surfaces, or to human VEGF-A associated or close to human cells that produce it, among the latter those present in human tumors growing in nude (athymic) mice.

The recombinant polypeptide molecules described in the present invention scFv 2H1, Fab 2H1-32, scFv₂-Fc 2H1-4.1 and scFv₂-Fc 2H1-8.2, interact with human active VEGF-A in a way such that interferes with its growth promotion activity in human endothelial cells *in vitro*, and in *in vivo* neo-angiogenesis processes, measured the latter in the experimental models of subcutaneous Matrigel pellets in mice, and of human tumor cells transplanted in syngeneic athymic mice (nude mice).

The recombinant polypeptide molecules described in the present invention do not recognize mouse VEGF-A, and effectively interfere in the association between human VEGF-A and a soluble form of the KDR receptor. The application of the polypeptide molecules scFv 2H1 and scFv₂-Fc 2H1-4.1 prevents choroideal neo vascularization (CNV), or improves its evolution, in an experimental model of non human primates where CNV is produced via photo-coagulation with laser. The polypeptide molecules Fab 2H1-32 and scFv₂-Fc 2H1-8.2 would also able to produce such effects on CNV, taking into account that their specificity for human VEGF is similar to that of the polypeptide molecules scFv 2H1 and scFv₂-Fc 2H1 4.1, and have anti-angiogenic effect in other *in vitro* and *in vivo* models.

The recombinant polypeptide molecules described in the present invention can be conjugated to an enzyme or its fragments, to a biological response modifier, to a toxin or a drug, or to radioactive isotopes, that add to the original molecule a functional characteristic additional to that of binding to the human VEGF-A, being this the capacity of identifying and/or affecting the viability of cells that are located in a particular anatomical location of a multicellular organism where a high concentration of human VEGF-A exists, or in its immediate vicinity, or by interact ing with VEGF-A forms associated to the cell membrane.

The recombinant polypeptide molecules object of the present invention, inasmuch they posses the capacity of recognizing and interacting with human VEGF-A and interfering with its pro-angiogenic activity and the ability of stimulating the proliferation of endothelial cells, can also affect other biological functions described for human VEGF, as those in which the molecule acts in the negative regulation of the immune response (Chouaib S et al. 1997. Immunology Today 18:493-497).

A set of elements make the recombinant polypeptide molecules object of the present invention to be novel with respect to other antibodies and antibody fragments that neutralize human VEGF-A. Among these elements are the following:
(a) the base sequences that encode for the VRs that form the antigen binding sites of the polypeptide molecules object of the present invention have not been reported before and differ from those of other anti-VEGF-A antibodies. The CDR sequences of the VRs and in particular, that of the CDR3 of the heavy chain VR, differs notably from other previously reported that are rich in the aromatic aminoacids tyrosine and/or tryptophan, a fact that has been related to the recognition a particular epitope (Fellouse F.A. et al. 2004. PNAS 101:12467-12472). In the case of the polypeptide molecules described in this invention, the CDR3 of the heavy chain VR possess no tyrosines.
(b) the immunochemical specificity of the polypeptide molecules object of the present invention for human VEGF-A differs from that of human Fab fragments obtained from other libraries (Fuh G. et al. 2006. J. Biol Chem 281: 6625-6631), that additionally also recognize mouse VEGF-A.
(c) the polypeptide molecules described in this invention are very dependent for their recognition of human VEGF-A on its biologically active dimeric conformation, as shown by the loss of recognition detected after treating VEGF-A with reducing agents.
(d) the polypeptide molecules described in this invention recognize a conformational epitope on human VEGF, not previously described when compared with the epitopes that recognize other antibodies and antibody fragments that neutralize the biological functions of human VEGF-A (Muller Y et al. 1997. PNAS 94: 7192-7197; Muller AY. et al. 1998. Structure 6: 1153-1167; Schaeppi J.-M. et al. 1999. J Cancer Res Clin Oncol 125: 336-342; Fuh G. et al. 2006. J Biol Chem 281: 6625-6631; WO2005012359).

As shown in Example 9, the *in silico* analysis of the probable associations between the peptide sequence specifically selected by the scFv 2H1 antibody fragment from a 12-aminoacid combinatorial peptide library, and the known data of the primary and tertiary structures of human VEGF-A, all suggest that the antigen binding site of scFv 2H1 is interacting with a conformational epitope in the human VEGF-A molecule. The zone recognized in the tertiary structure of VEGF does not coincide with the epitopes described for other antibodies and antibody fragments that neutralize human VEGF-A, and is thus novel. This epitope definition opens also new possibilities of knowledge on the complex interaction between human VEGF-A and its receptor KDR, that remains yet unresolved.

The polypeptide molecules scFv 2H1, Fab 2H1-32, scFv₂-Fc 2H1-4.1 and scFv₂-Fc 2H1-8.2 described in the present invention, and their equivalent variants, are able to interact with human active VEGF-A and interfere with its effect of stimulating neo-angiogenesis. Because of this, these molecules are useful for the development of novel treatments for diseases in which an abnormal and excessive angiogenesis is present, as: (a) cancer (primary tumors and metastases), (b) eye diseases as age-related macular degeneration (wet variant), neovascular glaucoma, diabetic and newborn retinopathy, (c) acute and chronic inflammatory processes as asthma, respiratory distress, endometriosis, atherosclerosis and tissue edema, (d) diseases of infectious origin as hepatitis and Kaposi sarcoma, (e) autoimmune diseases as diabetes, psoriasis, rheumatoid arthritis, thyroiditis, and (f) other several diseases and states, such as organ transplant rejection, hemangioma, and angiofibroma.

Another aspect of the present invention is the use of the described molecules to produce pharmaceutical compositions for the inhibition and angiogenesis and the treatment of associated pathological conditions. Such treatment comprehends the administration of an effective amount of the molecules described in the present invention to a human being.

In an embodiment of the present invention, the recombinant polypeptide molecules that recognize the human VEGF are useful for the treatment of malignant neoplasic processes and their metastases. In a preferred embodiment, they are effective in the treatment of carcinomas, sarcomas and vascularized tumors. In Example 12 we illustrate how the application of the molecules described in the present invention had the effect of inhibiting the growth of a human carcinoma, transplanted in nude athymic mice. Some examples of tumors that can be treated using this strategy include (but are not limited to): epidermoid tumors, squamous tumors as those of head and neck, colorectal tumors, prostate, breast, lung (including small and non-small cell tumors), pancreatic, thyroid, ovary, and liver. The molecules should also be effective in the treatment of other types of tumors as Kaposi sarcoma, central nervous system neoplasia (neuroblastoma, capillary hemangioblastoma, meningioma, and brain metastases), melanoma, renal carcinoma, gastrointestinal tumors, rhabdomiosarcoma, glioblastomas, and leiomiosarcomas.

Because the recombinant polypeptide molecules related to antibodies described in the present invention exhibit an epitope recognition of human VEGF-A that distinguishes them from Bevacizumab, as shown in Example 5, and are thus different in the manner they interfere in the binding of human VEGF-A and its receptor, they can give rise to therapeutic effects *in vivo* that are different from other molecules that also act by inhibiting this interaction. It is well documented that it is possible to achieve different therapeutic effects *in vivo,* and different collateral effects of treatment, with antibodies produced against the same antigen but that recognize different epitopes, or that possess different affinity for the antigen (Allan D.G.P. 2005. The Oncologist 10: 760-761). It is also known by individuals skilled in the art that molecules that recognize human VEGF through regions different from those identified by the humanized antibody Bevacizumab, can produce effects of interference between VEGF and its receptor (Lee, F-H. et al. 2005. PNAS 102).

It is known that antibodies and antibody fragments as Bevacizumab and Ranibizumab have application in other diseases that progress with excessive angiogenesis (Gaudreault, J. et al. 2005. Invest Ophthalmol Visual Sci 46:726-733; Costa, RA et al. 2006. lnvestig Ophthalmol Visual Sci 47:4569-4578). In an embodiment of this invention, the described recombinant polypeptide molecules are useful for the treatment of the age-related macular degeneration, in its wet variant. Two of the molecules described in this invention, specifically scFv 2H1 and scFv₂-Fc 2H1-4.1, showed preventive and therapeutic effect (Example 14) on choroideal neo vascularization induced by laser burning in an experimental non human primate model. With respect to the basis of possible differences in therapeutic potential of the referred molecules with respect to those existent in the state of the art, we must underline the intrinsic differences in antigen recognition (a different epitope), as well as the fact that scFv 2H1 has approximately half the molecular size of the Fab fragment Ranibizumab, a fact that would allow better penetration of the retinal layers, an issue that has been stressed as important for a better therapeutic effects. Also, the scFv₂-Fc 2H1-4.1 molecule has a smaller molecular size than Bevacizumab, with similar potential advantages when compared in this case with the former.

In another embodiment of this invention, the described recombinant polypeptide molecules, or their equivalent variants, are used in *in vivo* diagnostic procedures for human cancers that express VEGF, as for example, colon, lung or breast adenocarcinomas, and others. For this, the polypeptide molecules specific for human VEGF-A described in this invention can be radiolabelled and injected in the form of agents that allow the production of images that demonstrate the presence and localization of tumors that express VEGF-A in man. For this, polypeptide molecules as those described in this invention are associated to a radioactive isotope and the binding of these to the tumor is assessed. The method can comprehend the administration of the radiolabelled molecule to a human being. As shown experimentally in the invention, the scFv 2H1 fragment radiolabelled with ¹²⁵I binds to the human VEGF-A expressed by human tumor cells transplanted to athymic nude mice, and accumulates specifically in the tumor area. The reactivity with the tissues that express abnormally high amounts of human VEGF-A can be detected with any appropriate method. When a radionuclide as ¹²⁵I, ¹¹¹In or ^{99m}Tc is employed to label the polypeptide molecules described in this invention, these localize preferentially in the tumor, and not in normal tissues and the presence of the radioactive labeling in the tumor tissue can be detected and quantitated using a gamma camera or a gamma counter. The quality of the tumor image obtained correlates directly with the signal:background ratio (Goldenberg DM. 1992. Int. J. of Biol. Markers, 7; 183-188). The experimental use of ¹²⁵I is exemplified in this invention but does not limit the potential use of other different radionuclides. If these radionuclides have therapeutic capacity, as ¹³¹I, ⁹⁰Y, etc., the radiolabelled polypeptide molecules described in this invention can deliver a beneficial therapeutic effect to the patient, due to their lodging in the anatomical area of a tumor that produces human VEGF-A, and its effects on tumor cells, on the cells that form the tumor blood vessels, as well as on other cellular elements that compose the tumor stroma and produce VEGF-A.

In agreement with the previous, and as suggested in the aforementioned Example, the recombinant polypeptide molecules described in the present invention, or their equivalent variants, coupled to other agents, can be the basis of therapeutic methods that comprehend their administration as medicaments or pharmaceutical compositions. These molecules, coupled chemically or through genetic engineering methods to therapeutic radionuclides, toxins, drugs, or biological response modifiers, can direct the therapeutic effect of the coupled elements to anatomical areas with an abnormally high concentration of human VEGF-A, as can be that of a tumor and its immediate vicinity, and exert a therapeutic effect. The amount to administer, frequency, and intervals of administration, all depend on the nature and severity of the disease and these decisions are the responsibility of specialists and other medical doctors, based upon what is already known in this technical field.

The compositions of the present invention can be administered in isolated form or in combination with other treatments, either simultaneously or sequentially, all depending on the disease to be treated. The pharmaceutical compositions comprehend, apart from the active ingredient, a pharmaceutically accepted vehicle, buffer, a stabilizer or pharmaceutical "carrier", or other materials well known to those skilled in the art. These materials are non toxic, do not interfere with the efficacy of the active ingredient, and their precise nature depends on the route of administration, being this oral, mucosal, or parenteral (for example, intravenous injection).

The molecules described in the present invention, or their equivalent variants, are produced by the expression of the nucleic acid that encodes them. In consequence, the nucleic acid sequences that encode any of these described polypeptide molecules, and the procedures for the expression of such nucleic acids, are also part of the present invention. In a preferred embodiment, the nucleic acid encodes mainly (while not exclusively) for the DNA base sequences that are described for the molecules scFv 2H1, Fab 2H1-32, scFv₂-Fc 2H1-4.1 and scFv₂-Fc 2H1-8.2.

To achieve the recombinant expression of the molecules described in the present invention, or their equivalent variants, appropriate vectors can be chosen or constructed, containing the regulatory sequences that each case requires, including promoter, terminator, enhancer, polyadenylation, marker genes, and other pertinent sequences. The vectors can be plasmids.

### BRIEF DESCRIPTION OF THE FIGURES

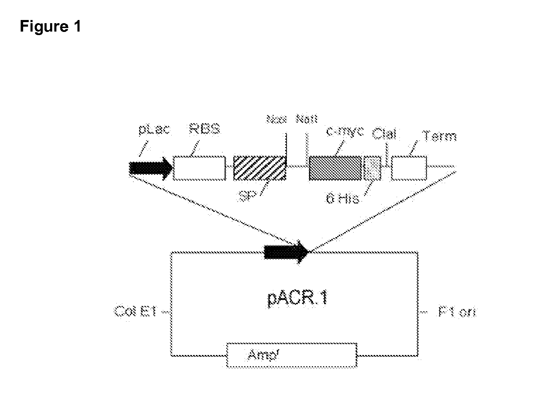

**Figure 1****.** Scheme depicting plasmid pACR.1, used in the production of soluble scFv fragments in the periplasm and culture medium of *E. coli.* The vector has a LacZ promoter, a Ribosome Binding Site (RBS) and the signal peptide (SP) *pe*/B.

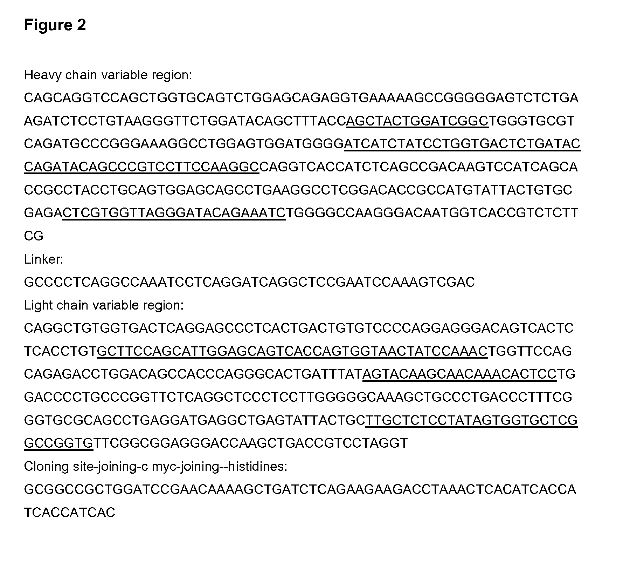

**Figure 2****.** DNA sequence (SEQ ID No.6) that encodes for the recombinant antibody fragment scFv 2H1, produced with the pACR.1 vector in *E. coli*. The first 354 bases conform the human immunoglobulin heavy chain variable region (VR) (denominated 2H1RVCP, SEQ ID No.7), followed by 48 bases that encode for the union segment (linker), continuing with 333 bases that encode for the human immunoglobulin light chain VR (denominated 2H1 RVCL, SEQ ID No.8), to end with 69 bases that encode for the aminoacids that are produced by the cloning site, the *c-myc* peptide, and six histidines encoded by the vector itself. Underlined bases represent the annotation of the Complementarity Determining Regions (CDRs), according to Kabat *et al.* (Sequences of Immunological Interest, Department of Health and Public Services, Fifth Edition, 1991), in the order (top to bottom): CDR1 of heavy chain VR (VH), CDR2 of VH, CDR3 of VH, CDR1 of light chain VR (VL), CDR2 of VL and CDR3 of VL.

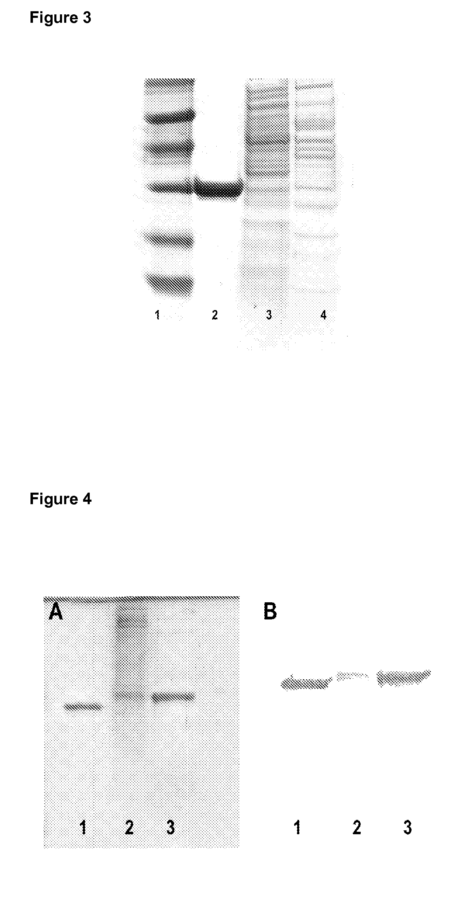

**Figure 3****.** Expression of scFv 2H1 in transformed *E. coli* BL-21 cells. From left to right: molecular weight markers (66, 45, 35, 29, 20 and 14.2 kDa; lane 1); a control scFv (scFv M3; lane 2), culture supernatant from bacteria transformed with plasmid pACR.1-scFv 2H1 (lane 3), where a reinforced band that migrates around 29 kDa can be seen, and culture supernatant from bacteria transformed with empty plasmid pACR.1 (lane 4).
**Figure 4****.** Results of purification of scFv 2H1 using IMAC. Figure 4A is a 12% Sodium Dodecyl Sulphate-Polyacrylamide Gel Electrophoresis (SDS-PAGE) with (left to right): control scFv (scFv M3) with molecular size around 29 kDa (lane 1), starting material that contains scFv 2H1 (lane 2), and scFv 2H1 eluted with high purity (lane 3). Figure 4B depicts a Western blot of a replica of the SDS-PAGE shown in Figure 4A. Monoclonal antibody 9E10 against the *c-myc* domain present in these proteins was used for detection.

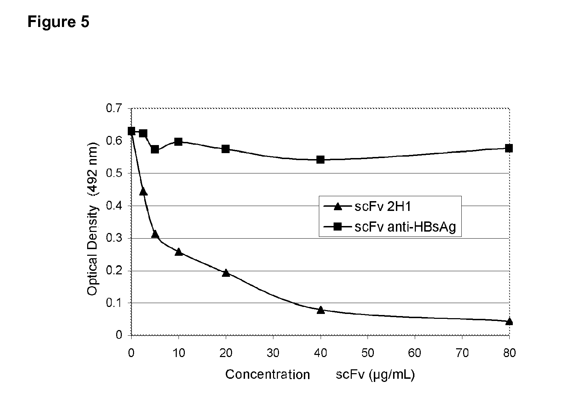

**Figure 5****.** Results of a competition ELISA where the capacity of purified scFv 2H1 to block the access of a soluble VEGF receptor molecule (KDR-Fc) to the ligand human VEGF-A was evaluated. VEGF-A was adsorbed to the solid phase. Different concentrations (up to 80 µg/mL) of scFv 2H1 were used. A scFv specific for Hepatitis B surface antigen (scFv anti-HBsAg) was used as negative control. In this assay, the detection of KDR-Fc bound to VEGF-A was done using peroxidase conjugated anti-human IgG antibodies (Sigma).

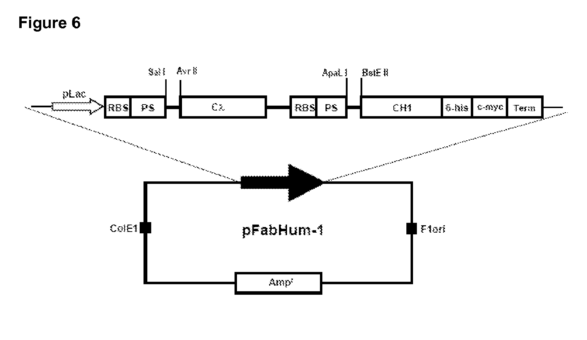

**Figure 6****.** Schematic representation of plasmid pFabHum-1, used for the production of soluble antibody fragments of the Fab type in the periplasm and culture medium of *E. coli.*

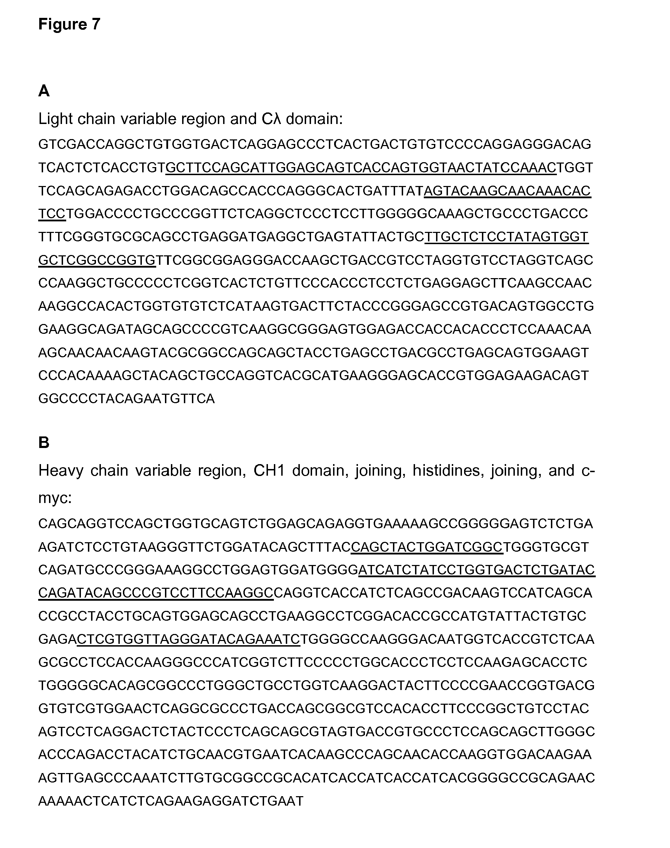

**Figure 7****.** DNA sequences (SEQ ID No. 9 and SEQ ID No. 10) encoding for the two chains that form the mature molecule of Fab fragment 2H1-32, that self-assemble in the bacterial periplasm. In Figure 7A and in the 5'-3' sense, the sequence encoding for the immunoglobulin light chain VR, followed by that encoding for the immunoglobulin Cλ constant domain, is shown. The underlined bases represent the CDR annotations in the order (top to bottom): CDR1 of the light chain VR (VL), CDR2 of VL and CDR3 of VL. In Figure 7B and in the 5'-3' sense, the sequence encoding for the immunoglobulin heavy chain VR, followed by that encoding for the CH1 immunoglobulin constant domain, six histidines and a *c-myc* peptide is shown. The underlined bases represent the CDR annotations in the order (top to bottom): CDR1 of the heavy chain VR (VH), CDR2 of VH and CDR3 of VH.

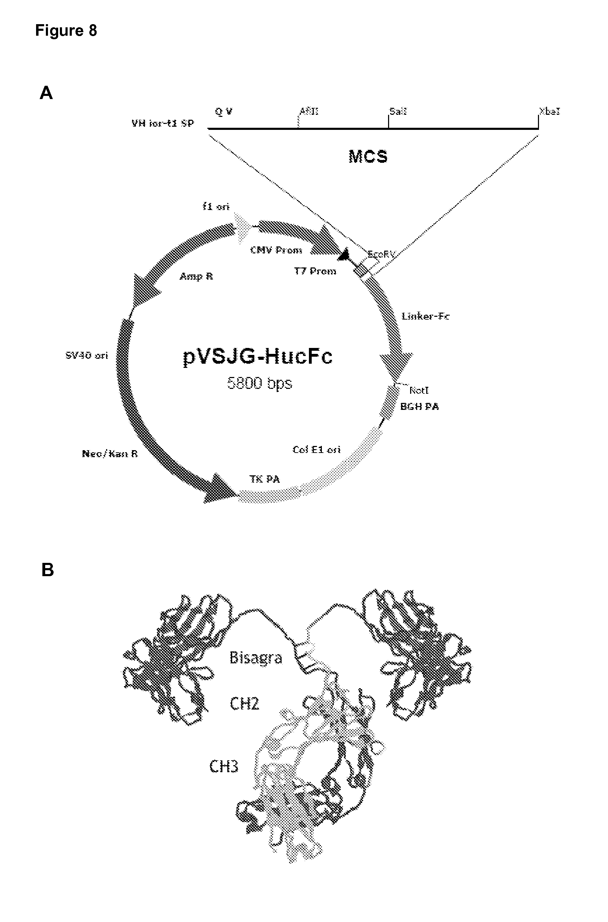

**Figure 8.** Figure 8A is the map of plasmid pVSJG-HucFc, used to express divalent molecules of the "full antibody" type, after cloning a scFv fragment between restriction sites Afl II and Xba I. Figure 8B is the schematic representation of the molecule produced by mammalian cells transfected with the plasmid containing a given scFv insert.

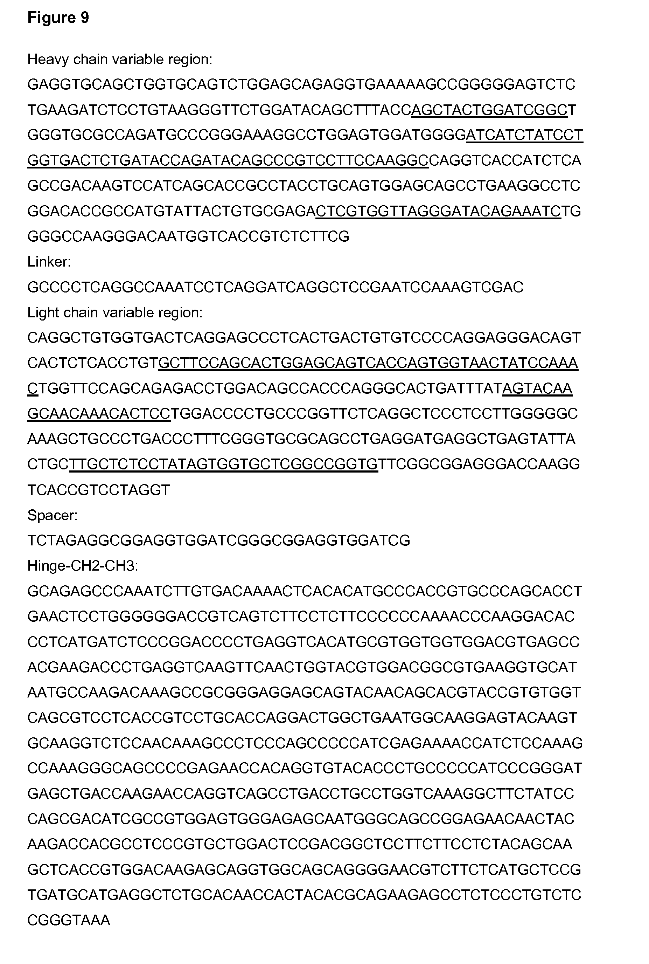

**Figure 9****.** DNA sequence (SEQ ID No. 13) that encodes for the mature antibody-like molecule denominated scFv₂-Fc 2H1-4.1. In the 5'-3' sense it can be seen: the bases encoding for the immunoglobulin heavy chain variable region, followed by a linker and the immunoglobulin light chain variable region, a spacer that encodes for 10 aminoacids, followed by the hinge, CH2 and CH3 domains of an IgG1 human immunoglobulin. The underlined bases represent CDR annotations in the order (top to bottom): CDR1 of VH, CDR2 of VH, CDR3 of VH, CDR1 of VL, CDR2 of VL and CDR3 of VL.

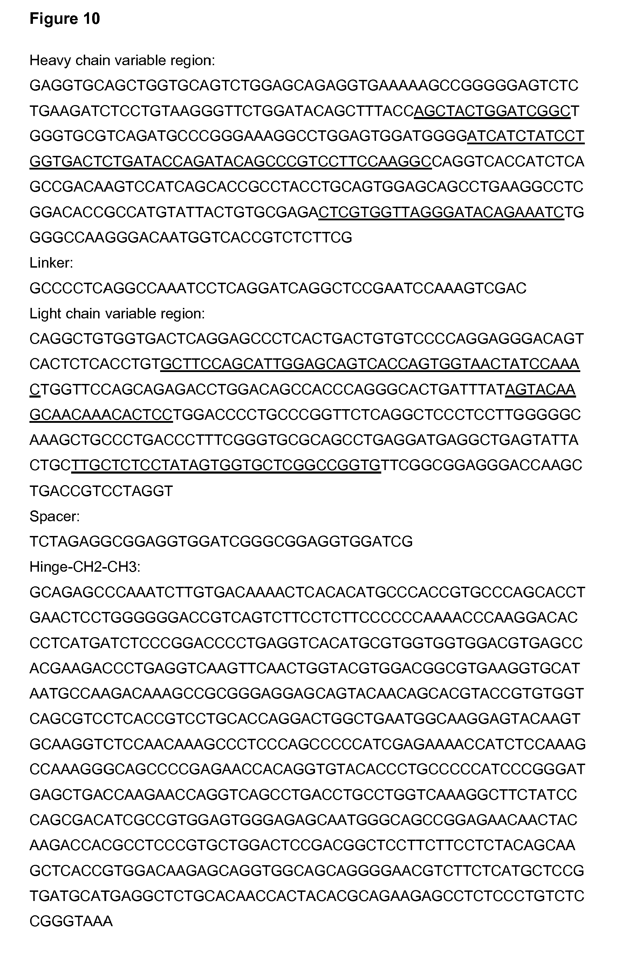

**Figure 10****.** DNA sequence (SEQ ID No. 14) that encodes for the mature antibody-like molecule denominated scFv₂-Fc 2H1-8.2. In the 5'-3' sense it can be seen: the bases encoding for the immunoglobulin heavy chain variable region, followed by a linker and the immunoglobulin light chain variable region, a spacer that encodes for 10 aminoacids, followed by the hinge, CH2 and CH3 domains of an IgG1 human immunoglobulin. The underlined bases represent CDR annotations in the order (top to bottom): CDR1 of VH, CDR2 of VH, CDR3 of VH, CDR1 of VL, CDR2 of VL and CDR3 of VL.

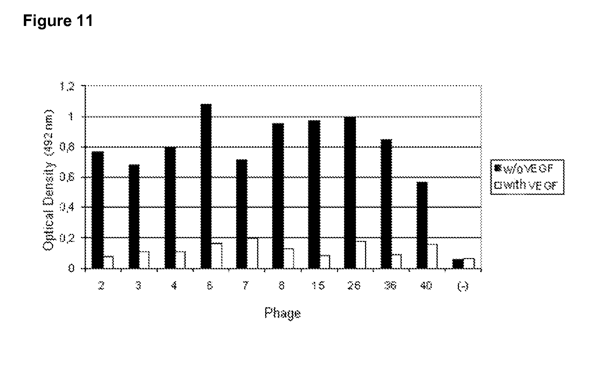

**Figure 11****.** Specific recognition of phage displayed peptides, in relation to the binding site of fragment scFv 2H1, as determined in an ELISA assay where the binding to the fragment adsorbed to a solid phase in the presence of excess human VEGF (Peprotech) was evaluated. The Figure shows 10 phage clones that display peptides that are representative of the behaviour shown by all 35 studied clones. In the experiment, the phage samples were incubated with or without (w/o) VEGF in solution.

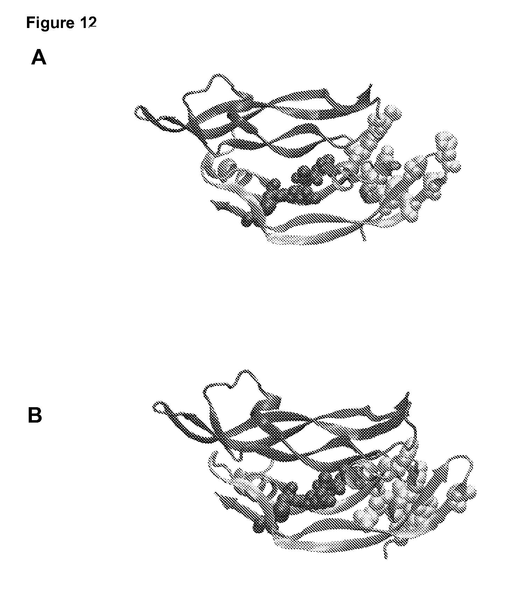

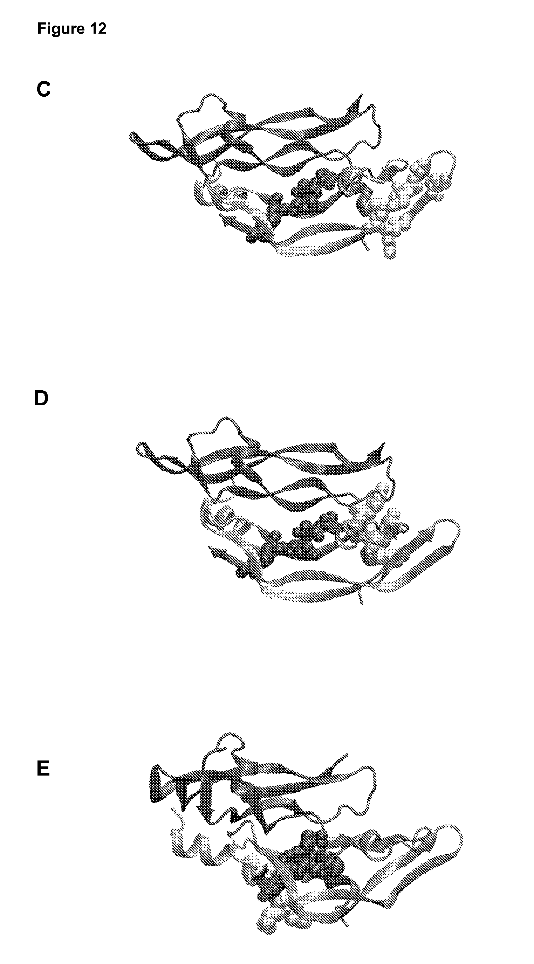

**Figure 12****.** Mapping of the residues that principally identify the epitope recognized by scFv 2H1 in the VEGF-A molecule, in comparison with other antibody-related molecules described in the literature. A diagram that represents the tertiary structure of human VEGF-A in its dimeric conformation is used, with alpha helix, beta chains and loops. The two identical dimer molecules appear in light grey and black. The positions of the residues defined as principal indicatives of the epitope recognized by scFv 2H1 are marked only in the light grey chain of the dimer, and appear in black, represented as Van der Waals (VDW) spheres. The residues recognized by other antibodies are depicted as light grey VDW in Figures A to D. Figure 12E shows the position of nearby aminoacids (in light grey VDW) that are different when the sequences of human and mouse VEGF-A are compared, in relation with the position of the epitope defined by the principal indicative residues for scFv 2H1 (in black VDW).

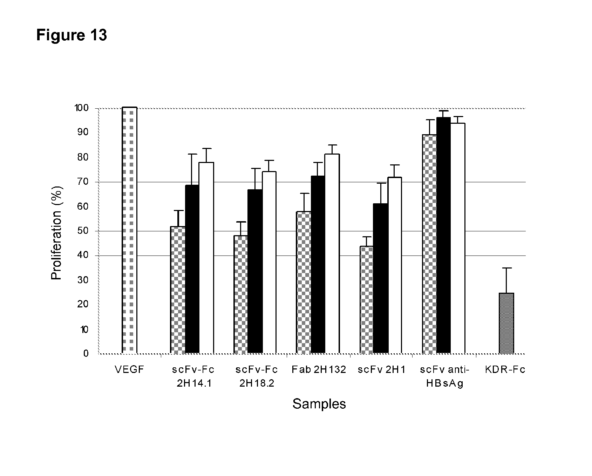

**Figure 13****.** Ability of the molecules scFv 2H1, Fab 2H1-32, scFv₂-Fc 2H1 4.1 and scFv₂-Fc 2H1 8.2 to interfere with the growth stimulatory effect of human VEGF-A on human umbilical cord vein vascular endotelial cells in culture (HuVEC). Purified scFv 2H1, Fab 2H1-32, scFv₂-Fc 2H1 4.1 and scFv₂-Fc 2H1 8.2 were added at three different concentrations (striped bar: 2 µg/mL; full bar: 1 µg/mL; empty bar: 0.5 µg/mL) with 10 ng/mL of human VEGF-A (Peprotech).

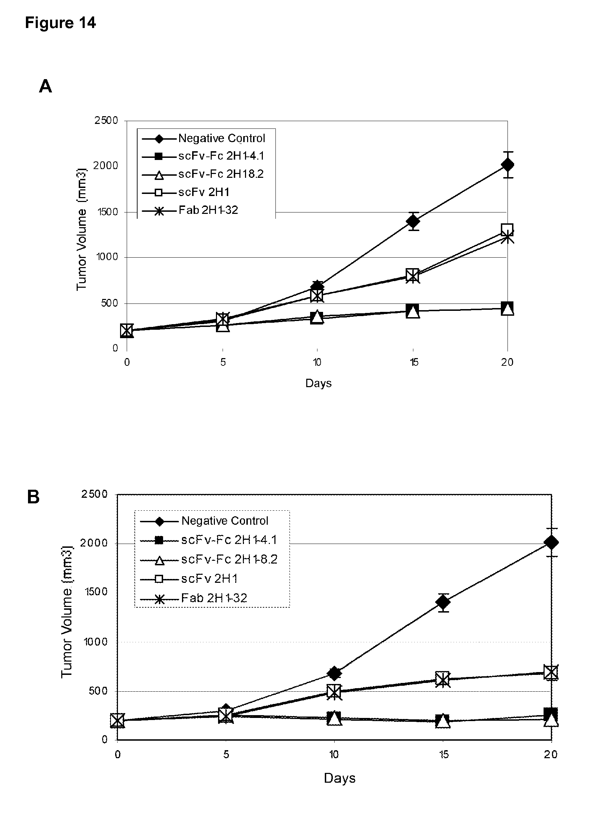

**Figure 14****.** Anti-tumor activity of scFv 2H1, Fab 2H1-32, scFv₂-Fc 2H1 4.1 and scFv₂-Fc 2H1 8.2 at doses of: 2.5 mg/kg (Figure 14A) and 25 mg/kg (Figure 14B). The points in the curves refer to the average of the tumor volume estimated for 5 animals per group. Negative control: unrelated monoclonal antibody CB-Hep.1 (anti-HBsAg) at the highest dose.

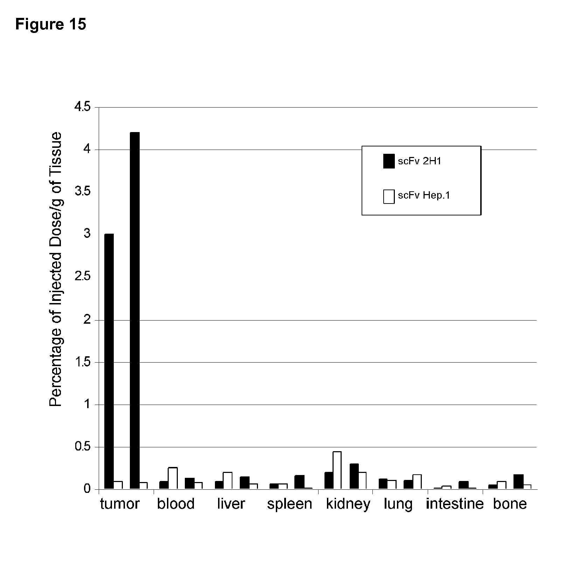

**Figure 15****.** Percentage of the injected dose, per gram of tiussue, 24 hours (first two bars in each tissue) and 48 hours (second two bars in each tissue) after inoculation of nude mice bearing human tumors derived from A431 cells with fragment scFv 2H1 (dark bars) or fragment scFv Hep.1 (empty bars) radiolabelled with ¹²⁵I. Each bar represents the average of the counts recovered from the organs/tissue obtained from 5 mice.

### Detailed description of the embodiments / Examples

### Example 1. Cloning, bacterial expression and purification of isoform 121 of human VEGF fused to P64K.

### (a) Cloning of isoform 121 of human VEGF

Polymerase chain reaction (PCR) was used for the isolation and cloning of isoform 121 of VEGF, employing Taq DNA Polymerase (Roche) and the procedure recommended by the manufacturer. Synthetic oligonucleotides # 1 (SEQ ID No. 1) and # 2 (SEQ ID No. 2) that appear in Table I, were used as primers in the PCR.

**Table 1.- Primers used in the PCR of isoform 121 of human VEGF.**

| **Oligonucleotide** | **Sequence** |
|---|---|
| # 1 (SEQ ID No.1) | 5’…GATCTGCTAGCCGCACCCATGGCAGAAGGAGGAGGG.. .3’ |
| # 2 (SEQ ID No. 2) | 5'...GGGGGATCCCCGCCTCGGCTTGTCAC...3' |

In this technique, the DNA template used was plasmid pVEGF (Ojalvo, A. G. et al. 2003. Electronic J. Biotechnol. 6, 208-222), previously modified by PCR to include mutations that substitute the VEGF aminoacid residues R82, K84 and H86, for glutamic acid.

The band corresponding to the amplification product was extracted from a 2% agarose gel. After band digestion with endonucleases Nhe I and BamH1, the DNA was purified and cloned in vector pM238 (Yero, D. et al. 2006. Biotechnol. Appl. Biochem 44:27-34). With this vector the proteins expressed in bacteria are fusion proteins with an N-terminal domain of 47 aminoacids of the P64K protein of *Neisseria meningitidis*. The resulting plasmid (P64₄₇ₐₐ-VEGF) was sequenced and determined to contain only the *exprofeso* mutations described above, as shown in SEQ ID No. 3, with respect to the aminoacid sequence reported for the corresponding cloned human VEGF isoform by the European Molecular Biology Laboratory (www.embl-heidelberg.de).

### (b) Expression of the fusion protein in E. coli

Plasmid DNA was extracted from clone P64₄₇ₐₐ-VEGF and used to transform *E. coli* BL21. Several resulting colonies were inoculated in 50 mL of LB medium with Ampicillin and Triytophan and grown for 5h at 37°C. Twenty-fine mL of these cultures were inoculated in 250 mL of enriched M9 medium with Ampicillin and incubated again for 2h at 37°C. The induction was done adding 40 µg/mL of 3-β-indoleacrylic acid. The cells were collected 8h later and after ultrasonic rupture, the protein of interest remained in the precipitate. Solubilization of the protein was done with sodium phosphate buffer plus 6M urea. The supernatant was subjected to IMAC in a Ni-NTA gel (QIAGEN).

The eluted fraction was evaluated using SDS-PAGE showing bands of 26 kDa, 54 kDa and higher, with the two first bands comprising more than 98% of the protein in the preparation. The first size corresponds to that of a monomer, the second to a dimeric molecule, and the higher weight ones to further aggregation. The elution fraction was dialyzed in PBS, subjected to chromatography in Superose F12 (Pharmacia), to select exclusively the species with higher apparent molecular weight (around 54 kDa) that were denominated P64₄₇ₐₐ-VEGF. This preparation was frozen for further use at-20°C.

### (c) Receptor binding assay of the fusion protein P64₄₇ₐₐ-VEGF.

A Maxisorp (Nunc) 96-well immunoplate was coated with the purified P64₄₇ₐₐ-VEGF fusion protein or human VEGF-A (Peprotech), both at 5 µg/mL in PBS for 16h at 4°C. The plate was blocked at 22°C with PBS-milk 4% during 1h. The soluble human receptor KDR-Fc (Sigma) was diluted in PBS-milk 4% at different concentrations, added to the plates, and incubated for 1h at 22°C. After washing repeteadly, the binding of the receptor to the coating was developed using goat anti human IgG antibodies conjugated with peroxidase (Sigma) followed by a substrate solution made of *orto*-fenilendiamine 0.5 mg/mL and hydrogen peroxide 0.015%, in citrate buffer pH 5.5. The plates were read in an ELISA plate reader at 492 nm, and the mean absorbace values estimated from three replica wells per experimental sample.

Table 1a shows that the binding of the soluble receptor was limited to the solid phase coated with human non mutated VEGF-A from Peprotech. The fusion protein P64₄₇ₐₐ-VEGF, in which three critical mutations were introduced by us purposedly (residues R82, K84, and H86 were substituted by glutamic acid) was not identified by the soluble KDR, a result that coincides with predictions made by Shen et al. (Shen, B. et al. 1998. J Biol Chem 273: 29979-29985), and makes our antigen different from others reported in the literature as used for the development of antibodies and antibody fragments that neutralize human VEGF.

**Table 1a. Binding assay for KDR-Fc, using VEGF-A or the fusion protein P64₄₇ₐₐ-VEGF as coating in ELISA.**

| KDR-Fc (µg/mL) | Coating of ELISA plate wells | |
|---|---|---|
| | Isoform 121 of human VEGF-A | P64₄₇ₐₐ-VEGF fusion protein |
| 0 (negative control) | 0.102 | 0.115 |
| 1.0 | 1.48 | 0.120 |
| 0.5 | 1.27 | 0.118 |
| 0.25 | 1.04 | 0.122 |
| 0.125 | 0.760 | 0.109 |

### Example 2. Immunization of BALB/c mice with the P64₄₇ₐₐ-VEGF protein and evaluation of the capacity of sera to recognize a commercial isoform 121 of human VEGF.

The potential of the fusion protein P64₄₇ₐₐ-VEGF to generate VEGF neutralizing antibodies was evaluated immunizing 10 BALB/c female mice (CENPALAB, Havana) between 8-10 weeks of age. The animals were immunized with 100 µg of the P64₄₇ₐₐ-VEGF protein per dose, using the adjuvant known as VSSP (Estévez F. et al. 1999. Vaccine 99: 190-197), in a scheme of four subcutaneous doses, spaced 7 days. A week after the last immunization the animals were bled and the serum separated, and stored in aliquots at -20°C.

To determine whether the sera from mice immunized with P64₄₇ₐₐ-VEGF had specific antibodies for VEGF an ELISA was developed coating with isoform 121 of human VEGF-A (Peprotech). The latter was immobilized in a 96-well Maxisorp (Nunc) immunoplate at a concentration of 1 µg/ml in PBS, during 16h at 4°C. The plate was blocked at 22°C with PBS-milk 4% during 1h. Serial serum dilutions in PBS-milk 4% were incubated for 1h, the plate washed, and incubated with an anti-mouse peroxidase conjugate (Sigma). The reaction was developed with a substrate solution made of *orto*-fenilendiamine 0.5 mg/mL and hydrogen peroxide 0.015%, in citrate buffer; pH 5.5. Serum of animals immunized with the P64₄₇ₐₐ-VEGF protein recognized specifically commercial human VEGF with titers up to 1:32,000.

### Example 3. Selection of antibody binding sites against human VEGF.

For the selection of binding sites against isoform 121 of human VEGF we used a filamentous phage display library of human single chain Fv (scFv) antibody fragments, constructed specifically for this invention. In this library, the human light chain variable regions (VR) present in the resulting scFv correspond to a lambda VR repertoire. In the process of developing this library biased towards lambda VR (Vλ), constructed according to published procedures (Rojas G, et al. 2005. Biochem Biophys Res Comun 336: 1207-1213), the genes encoding for a repertoire of human heavy chain VR were recovered from a semi-libray, and ligated to the plasmid DNA of another semi-library of Vλ regions at a 1:1 vector:insert ratio. The products of the ligation were electroporated into TG1 cells to obtain the final library. The presence and size of the inserts were determined in a sample of 30 colonies, with oligonucleotides that associate to the flanking regions of cloned scFv.

In the selection using this newly constructed library we employed as antigen the recombinant fusion protein P64₄₇ₐₐ-VEGF. The mixture of phage that conform the library was submitted previously to a depletion process with an excess (1 mg/mL) of P64k in solution, to eliminate the undesired scFv that are specific for this protein. The depleted mixture was used to probe the P64₄₇ₐₐ-VEGF protein immobilized in Maxisorp (Nunc) immunotubes. For this, the immunotubes were coated with 10 µg/mL of the protein in PBS, at 4°C overnight, and then blocked with PBS-skimmed milk 4%. The non-bound phages were eliminated through 20 washes with a solution of PBS-Tween 0.1%, followed by 2 washes with PBS. The bound phages were eluted with a 100 mmol/L triethylamine solution for 10 min that was immediately neutralized with 0.5 mol/L Tris (pH 7.5). The eluted phages were amplified in the TG1 *E. coli* strain and used as starting material for the next selection cycle. This procedure was repeated 3 times under the same conditions. Random individual colonies of TG1 cells infected with phages eluted from the second and third selection cycles were used to produce phages at 96-well scale. The capacity of these phage clones that display scFv antibody fragments to bind P64₄₇ₐₐ-VEGF was evaluated using ELISA. Maxisorp (Nunc) 96-well plates were coated with 10 µg/mL of P64₄₇ₐₐ-VEGF, and then blocked. The phages, diluted in PBS-skimmed milk 4% were incubated in the plates for 1h at 22°C, followed by several washes. The bound phages were detected with anti-M13 antibodies conjugated to peroxidase (Amersham) for 1 h at 22°C. Following several washes, the reaction was developed with the addition of substrate solution. The absorbance was read at 492 nm in a microplate reader. Of the 96 phage clones evaluated with the ELISA, 87 resulted positive. The DNA than encodes for the scFv antibody fragments that are displayed by the phage clones that resulted positive in ELISA was amplified by PCR and subjected to a restriction analysis with the enzyme BstN-I. The product of this digestion was checked in a 4% agarose gel. From this analysis we identified 7 different restriction patterns and a clone representative of each was selected. The selected clones were produced by infecting TG1 bacterial cells that were grown at 28°C for 16 h. The phages contained in the culture supernatants were precipitated with a solution of PEG 5000 in 2.5 M NaCl and stored in aliquots for the immunochemical characterization that follows.

### Example 4. Immunochemical characterization of the scFv in phage selected from the library.

### (a) Recognition of different isoforms of human and murine VEGF

To determine the specific recognition of isoforms 121 and 165 of human VEGF, and isoform 120 of mouse VEGF, the 7 phage clones that display scFv fragments were submitted to an ELISA assay. The immunoplates were coated with isoforms 121 and 165 of human VEGF-A (Peprotech) and isoform 120 of murine VEGF (R&D) at a concentration of 1µg/ml in PBS. After blocking the plates, the phages purified as described above and diluted in PBS-skimmed milk 4% were added and incubated for 1h at 22°C. After several washes, the bound phages were detected with anti-M13 peroxidase conjugated antibodies. The reaction was developed and quantitated as described in Example 2. As negative control, a sample of the mixture of phages of the unselected library was used, precipitated as described above. As seen in Table 2, the 7 selected phage clones identify specifically isoforms 121 and 165 of human VEGF. Of these, clones 2H1-F and 3C1 do not recognize isoform 120 of murine VEGF. The Table shows the recognition ability of the clones, that are classified as positive (+) when the obtained optical densities values in ELISA is at least 5 times that of the negative control.

**Table 2. Recognition of different isoforms of human and murine VEGF-A human by 7 clones of scFv displayed in filamentous phages.**

| **Phage clone** | **Isoform 121 of human VEGF-A** | **Isoform 165 of human VEGF-A** | **Isoform 120 of mouse VEGF-A** |
|---|---|---|---|
| 3C1 | + | + | - |
| 2B2 | + | + | + |
| 2D2 | + | + | + |
| 2E1 | + | + | + |
| 3E8 | + | + | + |
| 2H1-F | + | + | - |
| 2E3 | + | + | + |

### (b) Differential recognition of native and reduced human VEGF-A

The importance of the native homodimer folding of VEGF-A with respect to recognition by the different antibody fragments selected from the library was studied using an ELISA where isoform 121 of human VEGF (Peprotech) was coated to ELISA plates. After blocking the plates, half the wells were treated with a 50mM DTT solution in PBS-milk 4% during 1 h at 22°C and the other half with only PBS-milk 4%. After several washes, the wells where VEGF-A had been reduced with the DTT solution were treated with 100 mM iodoacetamide in PBS-milk 4% and incubated for 1h at 22°C. The remaining wells were maintained with PBS-milk 4%. After a new wash of the plate, the purified phage, diluted in PBS-milk 4%, were added to all wells and incubated for 1 h at 22°C. After several washes, bound phages were detected with anti-M13 peroxidase conjugated antibodies. As negative control, a sample of the mixture of phages of the unselected library was used. Table 3 shows 3 patterns: one in which the recognition was not affected by VEGF reduction (exemplified by clone 3C1), a second pattern in which a partial effect of reduction was seen (exemplified by clones 2B2 and 3E8), and a third pattern in which a complete abrogation of recognition against the reduced form of VEGF was noted (exemplified by clones 2D2, 2E1, 2H1-F and 2E3). The capacity of recognition of the clones for VEGF-A was measured in terms of the mean Optical Density (492 nm) of three replica wells in the experiment, taking as a lower reference that produced by the negative control.

**Table 3. Recognition of human VEGF-A treated or not with DTT by the 7 scFv clones displayed in filamentous phage.**

| **Phage clone denomination** | **Human VEGF-A with no treatment (native)** | **Human VEGF-A treated with DTT (reduced)** |
|---|---|---|
| 3C1 | 2.125 | 2.096 |
| 2B2 | 2.015 | 0.600 |
| 2D2 | 1.289 | 0.121 |
| 2E1 | 1.831 | 0.142 |
| 3E8 | 1.109 | 0.770 |
| 2H1-F | 1.250 | 0.103 |
| 2E3 | 1.803 | 0.125 |
| Unselected phage mixture | 0.122 | 0.103 |

### (c) Competition ELISA between a soluble form of the KDR receptor (KDR-Fc) and the selected phage for human VEGF-A

A competition ELISA was used to evaluate the capacity of the selected phage clones to block the access of a soluble VEGF receptor to the antigen. For this, Maxisorp (Nunc) 96-well immunoplates were coated with isoform 121 of human VEGF-A (Peprotech). The plates were blocked and further incubated with a mixture of the corresponding phage, diluted in PBS-milk 4%, with or without 2 µg/mL of soluble receptor (KDR-Fc, Sigma). Bound phages were detected using anti-M13 peroxidase conjugated antibodies (Amersham). As shown in Table 4, the clone that evidenced a higher blockade of KDR-Fc binding to VEGF-A was that denominated 2H1-F. The table shows the capacity of recognition of the clones for VEGF-A, measured in terms of the mean Optical Density (492 nm) of three replica wells in the experiment, taking as a lower reference that produced by the negative control.

**Table 4. Recognition of human VEGF-A after individually mixing or not 7 phage displayed scFv clones with 2 µg/mL of KDR-Fc.**

| **Phage clone denomination** | **With KDR-Fc 2** µ**g/mL** | **Without KDR-Fc** |
|---|---|---|
| 3C1 | 1.269 | 1.290 |
| 2B2 | 2.261 | 2.213 |
| 2D2 | 1.161 | 1.160 |
| 2E1 | 1.293 | 1.401 |
| 3E8 | 0.828 | 0.993 |
| 2H1-F | 0.577 | 1.149 |
| 2E3 | 0.884 | 1.088 |
| Unselected phage mixture | 0.121 | 0.115 |

### Example 5. Expression of the scFv 2H1 fragment in E. coli, purification, and characterization of its human VEGF recognition.

### (a) Cloning of scFv 2H1 in the pACR.1 vector and sequencing

The pACR.1 vector is a plasmid designed for the expression of antibody fragments in the periplasm of *E. coli* (Figure 1). As main elements of the vector we have the LacZ promoter, a signal peptide, the restriction sites NcoI and Not I for the insertion of the fragment gene, a domain encoding for the *c-myc* peptide and a sequence encoding for 6 histidines, the latter for the purification of expression products using IMAC. The phagemide DNA that contains the scFv denominated 2H1-F was used as template for PCR. This procedure was done with the enzyme ProofStart (Stratagene), under the instructions of the manufacturer. Synthetic oligonucleotides # 3 (SEQ ID No.4) and # 4 (SEQ ID No.5) were used as primers in the procedure (Table 5).

**Table 5. Synthetic oligonucleotides for the amplification and modification of the scFv 2H1-F contained in the phagemide vector, for its cloning in vector pACR.1.**

| **Oligonucleotide** | **Sequence** |
|---|---|
| #3 (SEQ ID No. 4) | 5'...CTATTCTCCCATGGCACAG...3' |
| #4 (SEQ ID No. 5) | 5'...TTCTGTATGAGGTTTTGC...3' |

A band of the expected size (700 bp) obtained after amplification, was purified from a 1% agarose gel using the QIAGEN DNA gel extraction kit, digested with Nco I and Not I (Promega) and re-purified for ligation. The pACR.1 vector was digested Nco I and Not I (Promega), and ligated with the digested band using T4 DNA ligase (Promega). The ligation product was used to transform *E. coli* competent cells (strain XL-1 Blue; Stratagene) by electroporation. The transformed cells were plated in selective solid medium and grown at 37°C. The employed methods are widely known (Molecular Cloning, A Laboratory Manual, Second Edition. 1989. Sambrook, Fritsch and Maniatis).

Plasmid DNA was purified from different colonies (QIAGEN MiniPrep kit), and checked by digestion for the expected ligation product with the already described restriction enzymes. Several plasmids were chosen to obtain the DNA consensus sequence of scFv 2H1, using automatic sequencing and specific primers that hybridize externally to the cloning regions of vector pACR.1. The DNA consensus sequence obtained for the complete fragment (VH-linker-VL-c *myc*-histidines) denominated scFv 2H1 (SEQ ID No. 6) is shown in Figure 2. The plasmid representative of this construction was denominated pACR.1-scFv 2H1. This Figure presents the individual sequences of the heavy chain VR (denominated 2H1RVCP; SEQ ID No. 7) and light chain VR (denominated 2H1RVCL; SEQ ID No. 8). According to the classification of Kabat *et al*. 2H1RVCP belongs to Subgroup I of human immunoglobulin VR, and 2H1RVCL can be classified in several groups of human immunoglobulin type lambda VR. Underlined in the Figure are the CDR sequences, annotated according to the Kabat *et al.* classification.

### (b) Expression of scFv 2H1 in E. coli

pACR.1-scFv 2H1 was used to transform competent BL21 *E. coli* cells. This strain allows the expression of the heterologous protein in the periplasm and/or culture medium. The transformation was plated in solid selective medium and allowed to grow at 37°C. A construction representative colony was grown in liquid medium and when 1.0 DO₅₃₀ₙₘ was achieved, induction was done for 12h using 1 mM de isopropyl-beta-D-thiogalactopyranoside (IPTG) in the culture medium. The cells were centrifuged and the periplasmic contents were isolated by osmotic shock and brief sonication. Both this periplasmic fraction and the culture supernatant were evaluated in 12% denaturing SDS-PAGE. This assay showed the expression of a protein of the expected apparent molecular weight over 29 kDa, when compared with the molecular weight markers and with a scFv against carcinoembryonic antigen [scFv-M3]; (Pérez L. et al. 2006. Biotechnol. Appl. Biochem. 43:39-48) (Figure 3). Most of the scFv 2H1 fragment is found in the culture supernatant.

### (c) Purification of the scFv 2H1 fragment using IMAC

The six-histidine domain present in the protein, provided by the pACR.1 vector, was used to establish the purification procedure. This sequence confer the proteins with a very high affinity for metal ions (Ex. Zn⁺², CU⁺² , Ni⁺²), that can be chelated to different chromatographic supports. The bacteria transformed with the pACR.1-scFv 2H1 vector were centrifuged, and the supernatant isolated, dialyzed for 72h in the coupling buffer (NaH₂PO4 50mM, 300mM NaCl, pH 7-8), and applied directly to Agarose-NTA (QIAGEN). Figure 4A shows that a high purity protein that migrates in the gel a little over 29 kDa is obtained after purification. The obtained fractions were submitted to Western Blot using a monoclonal antibody (9E10) specific against the *c-myc* peptide that is present in the scFv and in the control (scFv-M3), followed by rabbit anti-mouse IgG antibodies conjugated to peroxidase (Sigma). Figure 4B shows that the 9E10 monoclonal antibody detected the scFv 2H1 and that no important degradations are seen.

### (d) Specific recognition of human VEGF by scFv 2H1 in ELISA

Maxisorp (Nunc) 96-well immunoplates were coated with isoforms 121 and 165 of human VEGF-A (Peprotech) and isoform 120 of murine VEGF (R&D) at a concentration of 1µg/mL in PBS during 16 h at 4°C. After blocking the plates with PBS-skimmed milk 4%, the purified scFv 2H1 was added at different concentrations in PBS-skimmed milk 4% and incubated for 1h at 22°C. After several washes, a monoclonal antibody (9E10) specific against the *c-myc* peptide was added (1µg/mL), followed by rabbit anti-mouse IgG antibodies conjugated to horseradish peroxidase (Sigma). The binding of the fragment to the antigen in the solid phase was revealed and measured as described in other examples. An unrelated anti-HBsAgA scFv was used as negative control, obtained and purified in a similar fashion as described above for scFv 2H1. Table 6 shows that scFv 2H1 maintains the specific recognition of the original phage scFv (2H1-F). The Table shows the recognition capacity of the fragment for different VEGF-A, in terms of the mean Optical Density (492 nm) calculated from the values reported from three replica wells in the experiment, taking as a reference the value produced by the negative control.

**Table 6. Recognition of different isoforms of human and mouse VEGF-A by the fragment scFv 2H1.**

| **Fragment** | **Isoform 121 of human VEGF-A** | **Isoform 165 of human VEGF-A** | **Isoform 120 of mouse VEGF-A** |
|---|---|---|---|
| scFv 2H1 (20 µg/mL) | 1.193 | 1.199 | 0.110 |
| scFv 2H1(2 µg/mL) | 1.162 | 1.087 | 0.091 |
| scFv 2H1(0.2 µg/mL) | 0.753 | 0.612 | 0.086 |
| scFv anti-HBsAg (20 µg/mL) | 0.091 | 0.095 | 0.082 |

### (e) Competition ELISA between a soluble form of the KDR receptor (KDR-Fc) and the scFv 2H1 fragment for VEGF-A

A competition ELISA was used to evaluate the capacity of the purified scFv 2H1 fragment to block the access of a soluble VEGF receptor to the antigen. The assay is based on the inhibition of a soluble KDR-Fc receptor form to human VEGF-A coated to a solid phase, after the addition of increasing concentrations of scFv 2H1. For this, Maxisorp (Nunc) 96-well immunoplates were coated with isoform 121 of human VEGF-A (Peprotech) in PBS for 16h at 4°C. The plates were blocked and further incubated with increasing concentrations of purified scFv 2H1, and 0.5 µg/mL of soluble receptor (KDR-Fc, Sigma) or the vehicle alone (PBS-milk 4%). An anti-HBsAg scFv antibody fragment was used as negative control. Bound phages were detected using anti-M13 peroxidase conjugated antibodies (Amersham). The KDR-Fc bound to human VEGF-A in the solid phase was detected with anti-human IgG antibodies conjugated with peroxidase (Sigma). As shown in Figure 5 scFv 2H1 is capable of interfering the binding of the soluble receptor to human VEGF-A in the solid phase, with a clear dependence of the used dose.

### (f) Immunochemical comparison of scFv 2H1 and Avastin

The bacterial scFv 2H1 was compared with Bevacizumab (Avastin®, Genentech) with respect to their identification capacity of the fusion protein P64₄₇ₐₐ-VEGF, originally used for the antibody phage selection procedure described in Example 3 that gave rise to scFv 2H1-F. P64₄₇ₐₐ-VEGF or human VEGF-A (Peprotech) were immobilized in Maxisorp (Nunc) 96-well immunoplates at a concentration of 1 µg/ml in PBS, during 16h at 4°C. The plate was blocked at 22°C with PBS-milk 4% during 1h. Serial dilutions of purified scFv 2H1 or Bevacizumab in PBS-milk 4% were incubated for 1h, the plate washed, and further incubated as follows: (i) for scFv 2H1, with the 9E10 anti *c-myc* monoclonal antibody, followed by an anti-mouse IgG peroxidase conjugate (Sigma), and (ii) for Bevacizumab, with goat anti-human IgG antibodies conjugated to peroxidase (Sigma). The reactions were developed with a substrate solution made of *orto*-fenilendiamine 0.5 mg/mL and hydrogen peroxide 0.015%, in citrate buffer pH 5.5.

As seen in Table 6a, that depicts the mean absorbance values at 492 nm obtained in a ELISA plate reader, with three replica wells per studied sample, scFv 2H1 recognized both P64₄₇ₐₐ-VEGF and human VEGF-A, while Bevacizumab was only able to recognize human VEGF-A from Peprotech.

The unrelated scFv anti-HBsAg and TheraCIM (anti EGF receptor humanized IgG1 antibody; CIMAB SA, Havana) were used as negative controls.

**Table 6a. Immunoreactivities of fragment scFv 2H1 and Bevacizumab against P64₄₇ₐₐ-VEGF and isoform 121 of human VEGF-A.**

| **Antibody-related molecule** | **Isoform 121 of human VEGF-A** | **P64₄₇ₐₐ-VEGF** |
|---|---|---|
| scFv 2H1 (20 µg/mL) | 1.880 | 1.678 |
| scFv 2H1(2 µg/mL) | 1.480 | 1.113 |
| scFv 2H1(0.2 µg/mL) | 0.610 | 0.490 |
| Bevacizumab (20 µg/mL) | 3.201 | 0.145 |
| Bevacizumab (2 µg/mL) | 2.907 | 0.127 |
| Bevacizumab (0.2 µg/mL) | 1.885 | 0.110 |
| scFv anti-HBsAg (20 µg/mL) | 0.067 | 0.085 |
| TheraCIM (20 µg/mL) | 0.097 | 0.112 |

### Example 6. Expression of scFv 2H1 in Pichia pastoris and demonstration of its recognition for human VEGF.

### (a) Cloning of the scFv gene in the pPS9 vector

The gene that encodes for scFv 2H1 was digested NcoI/XbaI from the pACR.1-2H1 vector for cloning in the *Pichia pastoris* expression vector pPS9. Plasmid pPS9 is an integrative vector that contains a 1.15 kb fragment that corresponds to the promoter of the enzyme alcohol oxidase (AOX.1), followed by the gene that encodes for the secretion signal of sucrose invertase (sucll) of *Saccharomyces cerevisiae*, a multiple cloning site, a 960 bp fragment corresponding to the enzyme glyceraldehide 3-fosfate dehydrogenase (Gapt) for transcription termination, and the HIS3 gene of *S. cerevisiae* as selection marker. This vector also contains a 2.1 kb fragment that corresponds to the 3' sequence of the AOX.1 gene. All these elements are inserted in a pUC18 vector (EP0438200A1).

After NcoI/XbaI digestion of the scFv-2H1 gene and its purification from agarose gels, the resulting sequence was ligated to pPS9, previously digested Ncol/Spel, and the ligation products used to transform the XL-1 Blue *E*. *coli* strain. Isolated transformed colonies were analyzed using colony PCR with a primer that hybridizes in the promoter region, and those that contained the insert were selected. Sequencing was done as reported in other examples. The sequences obtained for the recombinant plasmids denominated pPS2H1-12 and pPS2H1-13 are identical, and contain that of scFv 2H1, reported in SEQ ID No. 6.

*P. pastoris* recombinant strains were obtained with these two plasmids (previously digested with Pvull [Promega]), using electroporation and the wild type strain MP36 his 3 (Yong V. et al. 1992. Biotechnol. Applic. 9: 55-61) and minimum medium deficient in histidine for selection. Due to the different recombination mechanisms of the plasmids with specific sites in the genome of *P. pastoris*, we isolated two different phenotypes of secreting cells: (a) strains where the AOX.1 gene was not affected during recombination and are able to grow in methanol and show similar behaviour to the wild-type strain (Mut+), and (b) strains where the AOX.1 gene was substituted by the expression cassette and showed slow growth in the presence of methanol (Mut s).

### (b) Expression studies

The expression studies for the antibody fragment were done starting from prototrophic His⁺ colonies grown in selective medium plates. The colonies were grown in 10 mL of rich buffered medium in 50 mL tubes, at 28°C and under 150 rpm. When the cultures achieved 2 D.O at 600 nm they were centrifuged at 2000 rpm, for 10 min. The cell pellets were resuspended in 10 mL of rich medium with methanol instead of glycerol as only carbon source. From that moment on and for the following 96 h, induction of the proteins of interest was done with daily additions of pure methanol up to 1% to the culture. The MP36his3 strain transformed with an empty vector was used as negative control.

When induction ended, the cells were centrifuged and the metabolized medium collected, centrifuged again for final clarification and 15% SDS-PAGE employed to detect scFv 2H1. This assay revealed the presence in both cases of proteins with the expected apparent molecular weight (29 kDa), that were later evaluated by Western Blot using monoclonal antibody (Mab) 9E10, as primary antibody, followed by rabbit anti-mouse IgG antibodies conjugated to peroxidase (Sigma). The two recombinant proteins were identified by Mab 9E10.

### (c) Recognition of human VEGF-A in ELISA by scFv 2H1

An ELISA assay similar with respect to solid phase, reagents, coating, incubation, development and control conditions to that described above for the *E*. *coli* derived scFv 2H1 was used. The culture samples of metabolized medium of the induced recombinant yeast strains diluted on PBS-1% milk were added and incubated 2h at room temperature. As negative controls the metabolized media of wild-type strain MP36 his 3, and the unrelated scFv anti-HBsAg fragment were used. As positive control, the bacterially derived purified scFv 2H1 fragment was used. Absorbance values at least 4 times higher that those produced by the negative controls were considered positive. The samples of the metabolized medium with the scFv 2H1 fragment, denominated scFv 2H1-Pp17, produced after induction of transformed *P. pastoris* yeast cells were positive with respect to their capacity of recognizing human VEGF-A bound to a solid phase.

### Example 7. Obtention of bacterial Fab fragments using the variable regions (VR) of scFv 2H1 and characterization of its recognition of human VEGF. (a) Cloning of the VR of scFv 2H1 in the pFabHum-1 vector and sequencing

Figure 6 is a scheme of plasmid pFabHum-1, used for the production of Fab type antibody fragments in the periplasm and culture medium of *E. coli.* The vector has a LacZ promoter, a RBS, the sequence for a signal peptide (PS), sites for the cloning of the light chain variable region (VR) (Sal I and Avr II), the sequence encoding for a human immunoglobulin CA domain, followed by another RBS and PS sequence, sites for the cloning of the heavy chain VR (Apa LI and Bst EII) followed by the sequence encoding for a human immunoglobulin CH1 domain, extended to include the first cisteine of the human IgG1 hinge region. The VR-CH1 protein is expressed associated to a six-histidine domain for IMAC purification and a *c-myc* peptide for analytical purposes, both in its C-terminus, and provided by the vector.

The DNA corresponding to the phagemid that bears the scFv denominated 2H1-F was first digested with Sal I and Avr II to obtain the light chain VR. After checking its size in a 1.5% agarose gel, cloning was done in pFabHum-1. Once verified the cloning using restriction enzymes, the plasmid (denominated pFab-Hum-1 RVL) was replicated, purified, and subjected to a new digestion with Apa LI and Bst EII. In the case of Bst EII, digestion was partial. Once the size of the band was verified in 1.5% agarose, cloning was done in pFab Hum-1 RVL. The cloning was verified with restriction enzymes and the plasmid denominated pFab 2H1-32. This plasmid was replicated, purified and submitted to automatic sequencing. The DNA sequence encoding for the mature protein Fab 2H1-32 is shown in Figure 7. Figure 7A shows that of the combination of light chain VR and CA (SEQ ID No.9) and Figure 7B shows that of the combination of heavy chain VR and CH1 (SEQ ID No.10). The CDRs appear underlined, annotated according to the classification of Kabat *et al.*

### (b) Expression of the Fab in E. coli

Plasmid pFab 2H1-32 was used to transform *E*. *coli* BL21 competent cells. The transformation was plated in solid selective medium and growth allowed to proceed at 37°C for 16h. A representative colony was grown in liquid medium and after achieving 1 D0₅₃₀ₙₘ the culture was induced for 12 h with 1 mM IPTG. The cells were centrifuged and the periplasmic content isolated with osmotic shock and brief sonication, and both the periplasmic fraction and the supernatant analyzed in 12% SDS-PAGE. This assay revealed the presence of a protein of the expected size (aprox. 50 kDa), that was later evaluated by Western Blot using as primary antibody a monoclonal antibody (9E10) against the *c-myc* peptide, followed by rabbit anti-mouse IgG antibodies conjugated with peroxidase (Sigma). Western blot showed that the 9E10 antibody detected a protein of the expected size, both in the culture supernatant and in periplasm samples.

### (c) Purification of Fab 2H1-32 using IMAC and characterization of its VEGF recognition by ELISA

The recombinant bacteria produced by the transformation with pFab 2H1-32 were centrifuged and the supernatant dialyzed for 72h against the coupling buffer. The preparations containing the Fab were directly applied to Agarose-NTA (QIAGEN). After washing to eliminate *E*. *coli* contaminants, Fab 2H1-32 was obtained in the elution fraction with purity close to 85%, estimated using 12% SDS-PAGE.

The purified Fab fragment was evaluated for its capacity of recognizing human VEGF using an ELISA assay. Maxisorp (Nunc) 96-well plates were coated with isoforms 121 and 165 of human VEGF-A (Peprotech) or isoform 120 of mouse VEGF-A (R&D) at 1µg/mL. The purified Fab fragment was diluted and incubated for 1 h at 22°C. After washing, the anti *c-myc* peptide monoclonal antibody was added (1 µg/mL), followed by rabbit anti-mouse IgG antibodies, conjugated to peroxidase (Sigma). An anti-HBsAg scFv was used as negative control, and the bacterial purified scFv 2H1 used as positive control. As shown in Table 7 the Fab produced in *E. coli* recognizes specifically human VEGF in ELISA. The Table shows the recognition capacity of Fab Fab 2H1-32 fragment for the different VEGF-A isoforms, in terms of the mean Optical Density (a 492 nm) value obtained from three replica wells, taking as a reference the values produced by the negative control.

**Table 7. Recognition of different isoforms of human VEGF-A and mouse VEGF-A by Fab fragment 2H1-32.**

| **Fragment** | **Isoform 121 of human VEGF-A** | **Isoform 165 of human VEGF-A** | **Isoform 120 of mouse VEGF-A** |
|---|---|---|---|
| Fab 2H1-32 (20 µg/mL) | 1.020 | 1.101 | 0.089 |
| Fab 2H1-32 (2 µg/mL) | 1.087 | 1.065 | 0.090 |
| Fab 2H1-32 (0.2 µg/mL) | 0.675 | 0.645 | 0.070 |
| scFv anti-HBsAg (20 µg/mL) | 0.087 | 0.078 | 0.083 |
| scFv 2H1 (2 µg/mL) | 1.098 | 1.076 | 0.082 |

### Example 8. Production and recognition characterization of dimeric molecules that comprehend 2 scFv units genetically fused to a human IgG1 Fc.

### (a) Transfectomas producing anti-VEGF scFv₂-Fc molecules

To obtain "full antibody" type molecules with two identical binding sites as defined by scFv 2H1, a PCR was done using as template the plasmid pACR.1-scFv 2H1 that contains the gene sequence of scFv 2H1, and primers # 5 (SEQ ID No. 10) and # 6 (SEQ ID No.11) that appear in Table 8, in order to modify the DNA sequence that encodes for the antibody fragment and make it compatible for the cloning that follows. This procedure was done with PanoTaq (Panorama Inc.), under the instructions of the manufacturer.

**Table 8. PCR primers used to modify pACR.1-scFv 2H1 for its cloning in pVSJG-HucFc.**

| **Synthetic Oligonucleotide** | **Sequence** |
|---|---|
| #5 (SEQ ID No. 11) | 5'... ACAGGGCTTAAGGAGGTGCAGCTGGTGCAGTCTGG... 3' |
| #6 (SEQ ID No. 12) | 5'... TGTTGTTCTAGAACCTAGGACGGTGACCTTGGTCCC... 3' |

The amplified DNA was cloned in vector pVSJG-HucFc. This vector is depicted in Figure 8A and was designed for mammalian cell expression of a polypeptide chain that comprehends, in this order: a leader sequence (signal peptide) of the heavy chain of a murine monoclonal antibody, followed by a scFv fragment, a 10 aminoacid spacer, and the consesus sequences of the human IgG1 immunoblubulin hinge, CH2 and CH3 regions. The leader sequence directs the chain to the endoplasmic reticulum, where it dimerizes through the formation of disulfide bonds between the hinge regions, and the complementary association of CH2 and CH3 regions of two identical polypeptides. The dimerized hinge, CH2 and CH3 domains for a human immunoglobulin Fc, to whicn N-terminal segments two identical scFv convert this bivalent construction in a "full antibody" type molecule (Figure 8B). Among the main characterisitcs of this vector we find a Cytomegalovirus promoter.

The band corresponding to the amplification product was digested with Afl II an Xba I and cloned into pVSJG-HucFc, previously digested with the same enzymes. Of the bacterial colonies resulting from transformation, two were selected for sequencing. Automatic sequencing indicated that the two colonies produced almost identical recombinant plasmids that were denominated scFv₂-Fc 2H1-4.1 and scFv₂-Fc 2H1-8.2. The only difference in sequence among these two plasmids, is that the former has a heavy chain VR base that is different and "silent" with respect to the encoded aminoacid, and three light chain VR bases that are different, everything with respect to the VR sequences of scFv 2H1 (SEQ ID No. 7 and SEQ ID No. 8). The sequences that encode for the mature scFv₂-Fc 2H1-4.1 and scFv₂-Fc 2H1-8.2 proteins are shown in Figure 9 (SEQ ID No. 13) and Figure 10 (SEQ ID No. 14), respectively. In the Figure the CDR sequences are undelined, and annotated according to the classification of Kabat *et al.*

The two plasmids were purified under endotoxin-free conditions using the Pure Yield Plasmid Midiprep (Promega) system, and employed to transfect P3/x63.Ag8.653 myeloma cells using SuperFect (QIAGEN). Supernatants of the transfectomas developing from cells that were resistant to G418 were evaluated by ELISA. Maxisorp (Nunc) 96-well immunoplates were coated with isoform 121 of human VEGF (Peprotech). The supernatants of the transfectoma colonies were diluted in PBS-milk 2% and added to the plates and the presence of anti-VEGF molecules of the scFv₂-Fc type were detected with anti-human Fc antibodies conjugated to peroxidase (Sigma). The transfectoma cell colonies that secreted higher amounts of anti-VEGF scFv₂-Fc molecules, as detected by ELISA, were repeatedly cloned by limiting dilution in the presence of G418 and always evaluating the capacity of the selected clones to produce anti-human VEGF signals in ELISA. After two independient and consecutive clonings two stable clones were obtained, that produced scFv₂-Fc denominated scFv₂-Fc 2H1-4.1 and scFv₂-Fc 2H1-8.2.

### (b) Purification of scFv₂-Fc 2H1-4.1 and scFv₂-Fc 2H1-8.2, and evaluation of their capacity to recognize human VEGF in ELISA

Transfectoma clones producing the scFv₂-Fc 2H1-4.1 and scFv₂-Fc 2H1-8.2 molecules were cultured in 162 cm² flasks in the presence of 10% fetal bovine serum, and the supernatant collected after a high cell density was achieved. The supernatants were diluted 1:1 in 0.1 M sodium phosphate buffer, pH 7.0 and were purified independently through affinity chromatography, using Protein A Sepharose Fast Flow 4 (Amersham). The molecules scFv₂-Fc 2H1-4.1 and scFv₂-Fc 2H1-8.2 were eluted in glycine 0.2 M, pH 4.0 buffer and submitted to fast neutralization with 1 M Tris, pH 10.0. After dialysis against PBS, the concentration was calculated using UV absorbance at 280 nm and purity estimated by 12% SDS-PAGE. It was determined that the preparations were over 85% pure. The purified molecules were applied to an ELISA as described above, in comparison with unpurified supernatant,s showing that both purified preparations were able to recognize human VEGF-A.

### Example 9. Identification of the human VEG epitope recognized by scFv 2H1

### (a) Selection of peptides that are recognized by scFv 2H1 from a combinatiorial peptide library displayed in filamentos phage

For the identification of the human VEGF-A epitope recognized by scFv 2H1 we followed the strategy of facing scFv 2H1 to a combinatorial 12-aminoacid linear peptide library displayed in filamentous phage (Combinatoria Molecular. Santiago Vispo, N. (Ed.) Elfos Scientiae, 2004, La Havana). The phage mixture that composes the library, diluted in PBS-skimmed milk 4%, was added to immunotubes (Nunc, Maxisorp) where purified scFv 2H1 had been immobilized. The tubes had been coated with the scFv, and the solid phase further blocked with PBS-skimmed milk 4%. The phages that did not bind to the scFv in the immunotubes were eliminated with 20 washes of a PBS-0.1%Tween solution, followed by 2 washes with PBS. Bound phages were eluted with a 100 mmol/L triethylamine solution, which was immediately neutralized with 0.5 mol/L Tris (pH 7.5). The eluted phage were amplified in TG1 bacteria and used as starting material for a new selection cycle. This procedure was repeated 3 times under similar conditions. Random colonies of TG1 cells infected with phage eluted from the second and third selection cycles were used to produce phage at 96-wells scale.

The capacity of these peptide-displaying phage clones to bind to scFv 2H1 was evaluated using a phage ELISA. Maxisorp (Nunc) 96-well plates were coated with scFv 2H1 and then blocked. The phages produced by a single well were diluted in PBS-smikked milk 4% and incubated for 1 h and 22°C specific wells of the scFv 2H1 coated plates, followed by several washes with PBS-0.1 %Tween. The bound phages were detected using anti-M13 antibodies conjugated to peroxidase (Amersham). Of 40 phage clones assayed in ELISA, 35 resulted positive and were used in the methods that follows.

### (b) scFv 2H1 binding ELISA competion assay

To test if the selected 35 clones of phages displaying peptides specifically recognize the binding site of scFv 2H1, an ELISA was made where the competition of the peptides on phage with VEGF-A for the binding to scFv 2H1 coated to a solid phase. Maxisorp (Nunc) 96-well plates were coated with 10 µg per well of scFv 2H1 and then blocked. Half of the wells were incubated with 10 µg/mL of VEGF (Peprotech) in PBS-4% skimmed milk, and after several washes with PBS-0.1%Tween, the phage preparations were added diluted in PBS-4% skimmed milk containing 10 µg/mL of VEGF (Peprotech), that were incubated in conditions similar to those already described. In a simultaneous fashion, the rest of the plate was incubated with PBS-skimmed milk and after several washes with PBS-0.1%Tween, the phage preparations were added diluted in PBS-4% skimmed milk. Bound phages were detected using an anti-M13 antibody conjugated with peroxidase (Amersham). As seen in Figure 11 por the sample of the studied clones, the presence of VEGF completely interferes with the binding of the peptide displayed in phages to the immobilized scFv 2H1.

### (c) Peptide sequencing

A sample of 20 of the 35 peptide phage clones characterized in the above mentioned procedure was used to extract phagemid DNA for automatic sequencing. The sequences obtained for the 20 studied clones were identical, being these CCRTLMLLQYHR (SEQ ID No 15).

### (d) Predictive study of the epitope recognized by scFv 2H1

The sequence obtained above was analyzed using programs FINDEPI (http://www.biocomp.cigb.edu.cu/findepi) and 3D Epitope Explorer (Schreiber A. J. 2005. Comput Chem 26: 879-887), with similar results. The previous are computational methods that predict surface protein patches implicated in protein-protein interactions. As input data, the methods use the 3D structure of one of the elements in the interaction couple (template protein; in our case the human VEGF-A from the PDB protein data bank, code 1BJ1), and the aminoacid sequence of the second element in the interaction (binding protein; in our case the peptide CCRTLMLLQYHR; SEQ ID No. 15). As an example, the FINDEPI program generates a database of potencial mimotopes of each surface patch in the template protein, applying a number of stereochemical rules. This database is explored using profile alignment methods to detect mimotopes potencially similar to the peptide selected experimentally due to its binding. After applying a grouping algorithm, the program reports a list of exposed residues in the surface of the template protein, with possibility of being localized in the interaction interphase of the two proteins. The certainity of the method has been assessed with protein-protein complexes for which crystalographic structure is known, and for which experimental data are available on the sequence of the peptides that are bound by these molecules.

Both used programs define a similar interaction zone in the human VEGF-A molecule that comprehends mainly residues C102, C57, R56, T31 and L32 (annotated according to the PDB protein data bank, code 1BJ1). It is considered in this invention that these residues are principal indicators of the epitope recognized by scFv 2H1. According to the predictions, other residues could also appear associated to the principal indicator ones mentioned above, whereas with lower scores, being these G59, C68, V69, P70 and H99. The epitope principally defined through residues C102, C57, R56, T31 and L32 does not coincide with those reported for other antibodies and antibody fragments that neutralize human VEGF (Muller AY et al. 1997. PNAS 94: 7192-7197; Muller AY. et al. 1998. Structure 6: 1153-1167; Schaeppi J.-M. et al. 1999. J Cancer Res Clin Oncol 125: 336-342; Fuh G. et al. 2006. J Biol Chem 281: 6625-6631; WO2005012359). The epitope principally defined through residues C102, C57, R56, T31 and L32 can be related with the conservation of the dimeric structure of VEGF-A, if we consider the results of the experiments shown in Example 4, that indicate a loss of recognition of scFv 2H1 for human VEGF-A when the antigen is treated for reduction of the disulfide bonds, that separates dimers in monomers.

Figure 12 show the mapping of the residues that principally define the epitope recognized in the VEGF-A molecule by scFv 2H1, in comparison to those described for other antibodies. A *cartoon* type diagram representative of the tertiary structure of human VEGF-A is used, in its dimeric conformation, with alpha helix, beta chains, and loops. The two identical molecules in the dimer appear as light grey and black. To simplify, the position of the residues defined as principal indicators of the epitope recognized by the scFv 2H1 are only depicted in the light grey chain, and appear in black Van der Waals representation (VDW). The residues recognized by other antibodies are shown as light grey VDW in Figures 12A to 12D. Figures 12A and 12B show that the epitope defined by the principal indicative residues for scFv 2H1 are contiguous but not overlap to that defined by Fab G6 and B20-4 (Fuh G. et al. 2006. J Biol Chem 281: 6625-6631). Figure 12C shows that the epitope defined by the principal indicative residues for scFv 2H1 es very different and is structurally distant for that defined for the humanized antibody Bevacizumab, commercially known as Avastin. Figure 12D shows that the epitope defined by the principal indicative residues for scFv 2H1 does not overlap structurally with the epitope defined by antibody 3.2E3.1.1 (Muller AY et al. 1997. PNAS 94: 7192-7197).

Figure 12E shows the position of the contiguos aminoacids (in light grey VDW) that are different when the sequences of human and mouse VEGF-A are compared, in relation with the epitope defined by the principal indicative residues C102, C57, R56, T31 and L32 for scFv 2H1 (in black VDW). It is known in the state of the art that contiguous residues of a given epitope are critical for its tertiary structure projection, hence for its recognition by antibodies, and that the change of a single aminoacid is enough to determine the specificity of an antibody for a molecule of a given specie, in relation with that of another specie (Fuh G. et al. 2006. J Biol Chem 281: 6625-6631). These results can explain why the scFv 2H1 fragment does not recognize mouse VEGF-A.

All these elements indicate that the antigen binding site defined by the VR that compose the scFv 2H1 fragment is different from those of other antibodies and antibody fragments that neutralize human VEGF reported in the literature, not only with respect to aminoacid sequence, but also in terms of the epitope recognized in the antigen, and, in consequence, in its probable mechanism of interference with the biological functions of native human VEGF.

### Example 10. Evaluation of the in vitro anti-proliferative effect of different molecules that recognize human VEGF, in the model of human umbilical cord endothelial cells, stimulated with human VEGF.

The *in vitro* anti-proliferative effects of molecules scFv 2H1, Fab 2H1-32, scFv₂-Fc 2H1-4.1 and scFv₂-Fc 2H1-8.2 was determined in a model of human umbilical cord vein endothelial cells (HuVEC), stimulated with human VEGF. Briefly, 3,000 HuVEC cells (PromoCell GmbH) were plated per well of a 96-well culture plate (Costar), previously coated with 1% Gelatine (Sigma), in RPMI 1640 medium supplemented with 1% (v/v) fetal bovine serum (Gibco) an grown at 37°C in 5% CO₂ during 24 h. The cells were stimulated with fresh medium supplemented with 10 ng/mL of human VEGF-A (Peprotech) and incubated with different concentrations of the molecules scFv 2H1, Fab 2H1-32, scFv₂-Fc 2H1-4.1 y scFv₂-Fc 2H1-8.2.

Figure 13 shows the proliferation of HuVEC cells grown in the presence of 10 ng/mL of human VEGF-A, arbitraily defined as 100% (proliferation control with no interference, VEGF bar), and when mixtures of the purified molecules scFv 2H1, Fab 2H1-32, scFv₂-Fc 2H1 4.1 and scFv₂-Fc 2H1 8.2 were added to the cells at three different concentrations (striped bar: 2 µg/mL; full bar: 1 µg/mL and empty bar: 0.5 µg/mL), with 10 ng/mL of human VEGF-A (Peprotech). As inhibition control, the mixture of 0.5 µg/mL soluble receptor KDR-Fc (Sigma) was used. As negative control, a scFv anti-HBsAg was employed in the mixture. After 72 hours of incubation, the cells were stained with 0.5% crystal violet in 20% methanol. The plates were washed with water and air-dried. The staining was eluted with a 1:1 solution of ethanol in 0.1M sodium citrate and the absorbance read in a plate reader at 562 nm. The value of the basal proliferation absorbance was substracted to all plate values and the data were represented as the percentage of inhibition versus the maximum proliferation control. As shown in Figure 13, the molecules scFv 2H1, Fab 2H1-32, scFv₂-Fc 2H1 4.1 and scFv₂-Fc 2H1 8.2 inhibit the proliferation of HuVEC cells in a dose dependent manner, to values between 45 and 58%.

### Example 11. Evaluation of the in vivo anti-angiogenic effect of different molecules that recognize human VEGF in the subcutaneous Matrigel pellet model in mice.

The *in vivo* anti-angiogenic effect of molecules scFv 2H1, Fab 2H1-32, scFv₂-Fc 2H1-4.1 and scFv₂-Fc 2H1-8.2 was studied in the experimental model described by Passaniti et al. (Passaniti A et al. 1992. Lab Invest. 67:519-28). In this model, angiogenesis is induced through the subcutaneous inoculation of C57BI/6 mice with an extract of proteins of the extracellular matrix (Matrigel, Becton Dickinson) in the presence of pro-angiogenic factors. The animals were divided in groups of 10 and injected subcutaneously in the abdominal region with 500 µL of Matrigel containing 100 ng of human VEGF (Peprotech), and different concentrations of the molecules to assay, including an unrelated antibody (CB-Hep.1, anti-HBsAg, Heber Biotec, Havana). After six days the animals were sacrificed, the Matrigel pellets extracted, and the hemoglobulin contents of each determined by the Drabkin method using the Drabkin's reagent kit (Sigma) according to the manufacturer's instructions. Molecules scFv 2H1, Fab 2H1-32, scFv₂-Fc 2H1-4.1 and scFv₂-Fc 2H1-8.2 inhibit significantly (p<0.001) the vascularization induced by human VEGF in the Matrigel pellets, correlating this with the lowering of hemoglobulin contents.

### Example 12. Evaluation of the in vivo anti-angiogenic effect of different molecules that recognize human VEGF, in the experimental model of nude mice xenotransplanted with A431 human tumor cells.

Because the angiogenesis induced by the tumor and some tumor stroma cells is essential for their growth and dissemination, and this angiogenesis in mainly due to the VEGF produced by these cell elements, and effective model for the assay of anti-angiogenic substances is that of the inhibition of tumor growth in animals. Because scFv 2H1, and all the other molecules derived from its VR identify human and not mouse VEGF, the tumor growth model in mice was established with human tumor cells inoculated to isogenic athymic mice (nude mice; nu/nu). In the experiment, we used 9 groups of 5 nu/nu athymic mice of the BALB/c strain (CENPALAB, Havana), with 8-10 weeks of age. The treatment groups were organized for each of the four molecules to test (scFv 2H1, Fab 2H1-32, scFv₂-Fc 2H1-4.1 and scFv₂-Fc 2H1-8.2) considering two dose levels: 25 mg/kg and 2.5 mg/kg per mouse in PBS pH 7.2. The ninth group (negative control) was treated with the vehicle (PBS pH 7.2). Mice were injected subcutaneously with 5x10⁶ human A431 tumor cells (ATCC, CRL 1555) in the right dorsal zone. When the tumors achieved volumes of 200 mm³ mice were randomized in 9 groups of 5, and the treatment started as indicated for each experimental group. The administrations were done intraperitoneally, in a volume of 200 µL, every 2 days during 3 weeks. The control was inoculated with the un-related murine monoclonal antibody CB-Hep.1 at the highest dose. The follow up of tumor growth was done with measurements of the highest (length), and lowest (width) tumor diameters, using a digital caliper. The tumor volumes were calculated as: tumor volume (mm³) =0.52 x length (mm) x width² (mm). Tumor volumes along the observation period were compared using the one way ANOVA stadigraph and a Bonferroni post-test. After the established treatment period, the animals were sacrificed and the tumors were surgically removed and histologically analyzed using Hematoxiline and Eosine.

As shown in Figure 14, all animals inoculated withn scFv 2H1, Fab 2H1-32, scFv₂-Fc 2H1-4.1 and scFv₂-Fc 2H1-8.2 showed a stadistically significant reduction of tumor volume with respect to the negative control, and a marker dose dependency. Of the four molecules, scFv₂-Fc 2H1 4.1 and scFv₂-Fc 2H1 8.2 gave the best results, possibly this being related to the higher sizes of the molecules, that increases their bio-availability, even though that, in terms of number of binding sites per unit of mass, scFv 2H1 has a slight advantage over these molecules. The differences found for the Fab and the scFv 2 H1 fragments were not stadistically significant, even though molecular mass is almost double, in favor of the Fab. This could be due to the fact that, for antibodies that should neutralize soluble molecules, more than affecting directly tumor cells, tumor penetration (supposedly better with smaller size) is not as critical as bio-availability, that is only favores for the "IgG-type" molecules as scFv₂-Fc 2H1 4.1 and scFv₂-Fc 2H1 8.2 due to the presence of a Fc that is compatible for the recycling mediated by the FcRn (Vaccaro C. et al. 2005. Nature Biotechnol 23:1283-1288). For the latter property, the scFv and Fab fragments are not so different, as both lack Fc. The histological analysis showed that the treated tumors had a significant reduction in vascular density, a reduction in the diameter of blood vessels, an increase in tumor cell apoptosis, and a reduction in mitotic figures.

### Example 13. Capacity of the ¹²⁵I-radiolabelled scFv 2H1 fragment to lodge selectively in the tumor area using nude mice inoculated with A431 cells.

To determine the capacity of the scFv 2H1 fragment of lodging in the area where A431 cells were growing, this fragment, and a control one (a murine anti-Hepatitis B surface antigen scFv; scFv-Hep.1) were labeled with ¹²⁵I (Amersham, UK) using the lodogen procedure (Fraker PJ, Speck JC Jr. 1978. Biochem Biophys Res Comm 80:849-857) for final specific activities of 1.3 MBq/5 µg and 1.28 MBq/5 µg, respectively.

The radiolabelled products were analyzed in thin layer chromatography to detect incorporation into protein, reporting values of 93 and 95% of radiactivity, respectively. The capacity of the radiolabelled products to detect their corresponding antigens (human VEGF and HBsAg) was studied in a system where polystyrene immunotubes were coated with isoform 121 of human recombinant VEGF (5 µg/mL; Peprotech), or recombinant HBsAg (5 µg/mL; Heber Biotec, Havana), that were then blocked, and placed in contact with samples of the radiolabelled fragments of the corresponding specificity, adjusted to the amounts that could be theoretically captured by the solid phase. After incubations and washings we determined that the solid phase was capable of binding 84 and 82% of radioactivity, for the scFv 2H1 and the scFv-Hep.1, respectively, showing that the radiolabelling procedure did not change importantly the biological activity of the fragments.

To study the biodistribution we used 20 nu/nu mice. The animals were inoculated subcutaneously with 5x10⁶ human tumor cells of the A431 culture line in the right dorsal zone. When the tumors achieved volumes of around 300 mm³ the animals were randomized in 4 groups of 5 animals and treatment started. Mice were injected by the tail vein with the radiolabelled product (10 with scFv 2H1 and 10 with scFv Hep.1), and sacrificed in groups of five, for each product, after24 and 48 h. Tumor, spleen, liver, kidney, intestine, muscle, bone marrow and blood samples were removed by surgery or sampled. The acummulation of radioactivity was expressed as percentage of the injected dose per gram of tissue. Calibration was done using a standard sample of the injected dose. Radioactivity was measured using a scintillation gamma counter.

Figure 15 shows the percentage of radioactivity recovered per studied tissue, at different times, with respect to the total injected radioactivity. Together with the results resumed in Table 9 for the specific case of tumor:blood radioactivity ratio, the experiment showed that from 24 to 48 hours after injection, the scFv 2H1 fragment localizes preferentially in tumor tissue, different to the unspecific scFv Hep.1.

**Table 9. Tumor:blood radioactivity ratio for nude mice transplanted with human tumor A431 cells that express human VEGF.**

| **Molecule** | **24 h** | **48 h** |
|---|---|---|
| scFv 2H1 | 33.3 | 30.0 |
| scFv-Hep.1 | 0.4 | 1.0 |

These results suggest that the scFv 2H1 fragment can localize specifically anatomical zones where a high local concentration of human VEGF exists, as in a A431 tumor, and is though useful for the specific delivery of different therapeutic products to this zone, such as a radioactive isotope, or eventually a toxin or a drug. The values correspond to 24 and 48 h after injection of different ¹²⁵I radiolabelled molecules to the animals. Each ratio was calculated starting from the mean values derived from tissues recovered fron 5 mice.

### Example 14. Prevention of experimental choroides neovascularization (CNV) in non human primates using the scFv 2H1 fragment and the bivalent molecule scFv₂-Fc 2H1-4.1.

As a model for experimental choroides neovascularization (CNV) we employed that reported by Krzystolik et al. (Krzystolik M.G., et al. 2006. Acta Ophthalmol, 120:338-346). Six cynomolgus monkeys (*Macaca fascicularis*, CENPALAB, Havana) were mantained and manipulated according to the Good Laboratory Animal Practice guidances of the institution. The animals were anesthesized for all procedures with intramuscular injections of ketamine hydrochlorate, acepromazine maleate, and atropine sulphate. Topical anestesia with proparacaine hydrochlorate was also used. Anesthesia befote enucleation and eutanasia was done with intravenous sodium pentobarbital. The CNV membranas were induced in the macula using argon laser burns, assuring the procedure produced a blister and a small hemorrage, with a point of application between 50 and 100 µm. Photography and fluorescent angiography were used to detect and measure the extension and characteristics of the lesions. The eyes of the animals were checked in different days, before and after application of the fragment and placebo and the laser burn procedure, as well as at the end of the experiment, that ended with enucleation and animal sacrifice.

The animals were divided in two groups of 3, according to the molecule to be studied: the scFv 2H1 antibody fragment or the bivalent molecule of immunoglobulin type scFv₂-Fc 2H1-4.1. The right eye of each animal received 500 µg of scFv 2H1 or scFv₂-Fc 2H1-4.1, according to the group, in 50 µL of PBS through intravitreous injection, while the left eye was only injected with the vehicle. The eyes received 2 injections befote laser treatment (days 0 and 14). On day 21, all eyes received the laser treatment for the induction of CNV. The injection was repeated in each eye in day 2 with the specific product or vehicle. Three weeks after laser induction (day 42), the animals received intravitreous injections, this time all with the scFv 2H1 fragment ot the scFv₂-Fc 2H1-4.1 molecules, according to the group, to end with a final similar injection on day 56.

In the phase I of treatment (before day 42), the studies showed a reduction in the onset of grade 4 lesions in the eyes where scFv 2H1 or scFv₂-Fc 2H1-4.1 were administered, in comparison with the respective control eyes, all of which suggests that the molecules help in the prevention of CNV. In the second phase of treatment, when all eyes received scFv 2H1 or scFv₂-Fc 2H1-4.1, we detected a reduction in grade 4 lesions that suggests that the fragment and the bivalent molecule are also beneficial for established lesions.

### Example 15. Expression of dimeric molecules that comprehend two units of the scFv fragment genetically fused to the Fc of a human IgG1 in transgenic tobacco plants.

PCR in conditions as described for Example 8 were used to amplify the gene that encodes for scFv₂-Fc 2H1-4.1, and modify the ends with the addition of appropriate restriction sites (Ncol and Xbal) to clone in plant cell vectors. The basic synthetic oligonulceotides used in PCR were designed over the sequences reported in SEQ ID No. 13. The amplified DNA fragment was detected as a majoritary band of approximately 1.4 kb and purified on 1% agarose gel (Sigma) using a QIAquick Gel Extraction Kit (QIAGEN, GmbH). The DNA was digested with the aforementioned enzymes and cloned in vector pHES74 (López A., et al. 1996. Biotecnología Aplicada 13: 265-270) in the form of a scFv-hinge-CH2-CH3 construction, preceeded of the signal sequence for sweet potato sporamine. This vector has the CaMV 35S promoter, a leader region of the omega tobacco mosaic virus that acts as translational augmenter for the amount of the produced protein, and a nopaline syntase terminator that also promotes the high expression or foreign genes in transgenic plants. The promoter-scFv-Fc-terminator gene expression "*cassette*" was introduced in the binary vector pDE1001 to produce the final plasmid pDEscFv-Fc.70. The details for the constructions used in this example are similar to those reported previously (Ramírez, N. et al. 2002. Transgenic Res. 11: 61-64).

The final plasmid pDEscFv-Fc.70 was used to transform *Nicotiana tabacum* cv. Petit Havana SR1 cells by gene transfer mediated by *Agrobacterium tumefaciens*. The F0 and F1 plants were obtained by conventional procedures previously described, and the expression of active scFv-Fc molecules was detected using an ELISA assay similar to that described in Example 8.

The biologically active scFv-Fc molecules are prepared in the form of total soluble plant proteins (TSP), extracted by grinding 0.4 g of transformed tobacco plant leaves, or untransformed controls, in liquid nitrogen until a fine powder is obtained, as described elsewhere. The powder is transferred to a reaction tube and mixed 1:2 (w/v) with extraction buffer (61 mM Tris-HCl pH 6.9; 2% SDS; 12.5% glycerol), and incubated in ice for 5 min. The insoluble material es removed centrifuging at 13,000 rpm and the soluble fraction assayed in ELISA at different dilutions, using anti-human Fc antibodies conjugated to alkaline fosfatase (Sigma) to detect expression. We detected that the TSP coming from transgenic plants contained molecules capable of recognizing human VEGF coated to a solid surface, that was not identified by the TSP originating from control plants.

## Claims

1. Recombinant antibodies **characterized in that** they comprehend human immunoglobulin variable regions encoded by nucleotide sequences SEQ ID No. 7 and SEQ ID No. 8, or homologous sequences that recognize an epitope in human VEGF-A defined in the context of, while not necessarily limited to, its residues C102, C57, R56, T31 and L32, and that interfere with its pro-angiogenic effect.

2. Recombinant antibody according to claim 1, **characterized in that** sequences SEQ ID No. 7 and SEQ ID No. 8, or homologous sequences, are comprised within the sequence encoding for a single chain Fv antibody fragment (scFv) where the heavy and light chain variable regions of human antibody origin are separated by a linker segment.

3. Recombinant antibody according to claim 2, where the scFv encoding sequence is SEQ ID No. 6.

4. Recombinant antibody according to claim 1, **characterized in that** sequences SEQ ID No. 7 and SEQ ID No. 8, or homologous sequences, are comprised within the sequence encoding for a Fab type antibody fragment, with constant domains of a consensus human IgG immunoglobulin.

5. Recombinant antibody according to claim 4, **characterized in that** the encoding sequences for the Fab type fragment are SEQ ID No. 9 and SEQ ID No. 10.

6. Recombinant antibody according to claim 1, **characterized in that** sequences SEQ ID No. 7 and SEQ ID No. 8, or homologous sequences, are comprised within the sequence encoding for a polypeptide chain, constituted by a scFv fragment linked by a spacer to human immunoglobulin hinge, CH2 and CH3 constant domains, that in its protein form covalently associates to another identical polypeptide chain to form a dimeric molecule.

7. Recombinant antibody according to claim 6, **characterized in** the human immunoglobulin constant domains are of the IgG1, IgG2, IgG3 or IgG4 types.

8. Recombinant antibody according to claims 6 and 7, **characterized in that** the sequences encoding for the polypeptide chain, constituted by a scFv fragment linked by a spacer to hinge, CH2 and CH3 constant domains, are SEQ ID No. 13 or SEQ ID No. 14.

9. Recombinant antibody according to claims 1 to 8, **characterized in that** it additionally contains a radioactive isotope, or a chemical or biological agent that possess anti-tumor or anti-angiogenic potential.

10. Recombinant antibody according to claims 1 to 8, **characterized in that** it additionally contains a radioactive isotope that confers potential for *in vivo* tumor diagnosis.

11. Recombinant antibody according to claims 1 to 8, **characterized in that** it is produced by recombinant bacteria or yeast, or in mammalian cells or other eukaryote systems.

12. Vectors that encode for recombinant antibodies according to claims 1 to 8, obtained through genetic manipulation via recombinant DNA, being these vectors plasmids or sequences capable of integrating into host cells.

13. Pharmaceutical composition that comprehends the recombinant antibody in claims 1 to 9.

14. Use of the recombinant antibodies of claims 1 to 9 for the manufacturing of a medicament for the treatment of entities that develop with an increase in angiogenesis, as eye entities, neoplastic processes, acute and chronic inflammatory processes, and autoimmune processes, through passive immunotherapy.

15. Use of the recombinant antibodies of claims 1 to 9 for the manufacturing of a medicament for the treatment of malignant tumors and metastases, through passive immunotherapy.

16. Use of the recombinant antibodies of claims 1 to 9 for the manufacturing of a medicament for the treatment of age-related macular degeneration, through passive immunotherapy.

17. Use of the recombinant antibodiy of claim 10 for the manufacturing of a radiopharmaceutical for the *in vivo* diagnosis of malignant tumors and its metastases, employing imaging techniques.
